# EUROPÄISCHE PATENTSCHRIFT

(11) **EP 2 996 796 B1**
(45) Veröffentlichungstag und Bekanntmachung des Hinweises auf die Patenterteilung: **09.12.2020**
(21) Anmeldenummer: 14723713.5
(22) Anmeldetag: 08.05.2014
(51) Int. Cl.: B01D 63/02, A61M 1/16, B01D 65/08

(54) **OXYGENATOR UND DESSEN VERWENDUNG**
OXYGENATOR AND ITS USE
OXYGÉNATEUR ET SON UTILISATION

(30) Priorität: 17.05.2013 EP 13002624
(43) Veröffentlichungstag der Anmeldung: 23.03.2016
(73) Patentinhaber: novalung GmbH, 74076 Heilbronn (DE)
(72) Erfinder: MAURER, Andreas, 72072 Tübingen (DE); BOGENSCHUETZ, Josef, 72406 Bisingen (DE); SCHMITZ-RODE, Thomas, 52070 Aachen (DE); STEINSEIFER, Ulrich, 4730 Hauset (BE); ARENS, Jutta, 52072 Aachen (DE); BORCHARDT, Ralf, 52066 Aachen (DE); SCHLANSTEIN, Peter Christian, 52070 Aachen (DE); WAGNER, Georg, 56651 Niederzissen (DE)
(74) Vertreter: Graf von Stosch, Andreas
(86) Internationale Anmeldenummer: PCT/EP2014/001245
(87) Internationale Veröffentlichungsnummer: WO 2014/183852

(56) Entgegenhaltungen:
- EP-A2- 0 521 495
- EP-A2- 1 864 709
- WO-A1-2014/085620
- DE-A1- 4 308 850
- US-A- 5 162 101
- US-A- 6 117 390
- US-A1- 2012 190 103
- US-A1- 2012 193 289
- US-A1- 2013 043 177

## Beschreibung

Die vorliegende Erfindung betrifft einen Oxygenator.

Als Oxygenatoren werden Blutgastauscher oder künstliche Lungen bezeichnet, die vorübergehend oder auch langfristig die natürliche Lungenfunktion ersetzen oder zumindest unterstützen sollen. Oxygenatoren werden z.B. auch auf dem Gebiet der Herzchirurgie als Teil einer Herz-Lungen-Maschine verwendet. Ein spezielles Einsatzgebiet ist die zumindest kurzzeitige Behandlung von akutem Lungenversagen. Oxygenatoren werden z.B. für Patienten eingesetzt, die insbesondere auf einer Intensivstation mit so genannter extrakorporaler Membranoxygenierung (ECMO) zur Herz- und/oder Lungenunterstützung therapiert werden. Der am weitesten verbreitete Typ von Oxygenatoren ist der Membranoxygenator. Oxygenatoren werden z.B. für die Abgabe von Sauerstoff an Blut und die Aufnahme von Kohlendioxid aus dem Blut eingesetzt. Hierzu kann ein sauerstoffreiches Gas z.B. durch semipermeable, nur gasdurchlässige Hohlfasermembranen geleitet werden, die von Blut umströmt werden. Ähnlich wie bei der natürlichen Lunge beruht der Gasaustausch dabei auf Diffusion, insbesondere aufgrund eines Konzentrationsunterschieds (einer Partialdruckdifferenz) von Sauerstoff bzw. Kohlendioxid in dem Blut und dem Gas.

Die Hohlfasermembranen bestehen z.B. aus einer Vielzahl von Hohlfasern aus mikroporösem Kunststoff, z.B. Polypropylen oder Polymethylpenthen, die in Lagen (insbesondere Fasermatten) angeordnet sind. Die einzelnen Hohlfasern weisen z.B. eine Länge von 100 bis 200 mm auf und sind z.B. in einem Abstand von 0,1-0,2 mm angeordnet. Sie können mit so genannten Kettfäden miteinander verbunden bzw. verknotet sein. Die durch eine Vielzahl von Hohlfasern gebildeten Fasermatten können einlagig oder doppellagig ausgebildet sein. Es können plasmadichte Hohlfasern mit geschlossener Außenschicht (geschlossen poröse Fasern, insbesondere Polymethylpenten (PMP)-basierte Fasern) oder nicht plasmadichte Hohlfasern mit nicht geschlossener Außenschicht (durchgängig poröse Fasern, insbesondere Polypropylen-basierte Fasern) vorgesehen sein. Ferner werden plasmadichte (insbesondere Polypropylen-basierte) und hochporöse, nicht plasmadichte (insbesondere Polymethylpenten-basierte) Fasern unterschieden. Die plasmadichten Fasern werden bevorzugt für Oxygenatoren verwendet, die über einen längeren Zeitraum von z.B. 14 Tagen, insbesondere bis zu 29 Tagen, eingesetzt werden müssen, insbesondere für die ECMO. Die Hohlfasern sind in einem Oxygenatormodul des Oxygenators angeordnet.

Nachteilig ist dabei insbesondere in Verbindung mit extrakorporalen Lungenunterstützungssystemen, dass die Oxygenatoren meist nicht länger als über einen Zeitraum von einem Monat eingesetzt werden können, insbesondere aufgrund der Gefahr von Koagulation (so genanntes "clotting"), was die Gasaustauschfähigkeit herabsetzt. Diese Gefahr besteht vor allem bei geringen Blutflüssen und/oder von sich aus geometrisch bedingt ungleichförmig durchströmten Oxygenatoren. Geringe Blutflüsse liegen z.B. im Bereich von 0.5-1.5 l/min oder darunter. Auch werden die Oxygenatoren insbesondere mit ansteigender Einsatzzeit nicht mehr gleichförmig durchströmt, also nicht mehr mit gleichförmiger Strömungsgeschwindigkeit, da sich Koagulationen bilden. Es entstehen häufig Bereiche, die nur mit geringerer als der optimalen Strömungsgeschwindigkeit durchströmt werden. Dies wirkt sich vor allem nachteilig auf die Gasaustauschfähigkeit von Sauerstoff (O₂) aus, wohingegen die Gasaustauschfähigkeit von Kohlendioxid (CO₂) stärker vom Gasfluss als vom Blutfluss abhängt. Hierbei besteht die Gefahr der Koagulation durch Areale, in denen eine Blut-Stagnation bzw. Blut-Stase auftritt.

Damit die Durchströmung mit Blut möglichst gleichförmig erfolgen kann, ist es bekannt, eine Blutverteilerplatte stromaufwärts von den Hohlfasern in einem Gehäuse des Oxygenators anzuordnen. Die Verteilerplatten haben die Aufgabe, den Blutstrom flächig aufzuspreizen. Sie können z.B. als gelochte, transparente Platten ausgeführt sein, welche im Bereich des auftreffenden Blutstroms keine Öffnungen aufweisen. Ferner kann auch ein Deckel des Gehäuses in einem Einlassbereich des Blutes eine die Strömung lenkende Geometrie aufweisen, und insbesondere bei einem eckigen Gehäuse eine schräge Ebene zum Ausgleichen von Druckunterschieden definieren. Hierbei werden auch Verteilerschenkel vorgesehen, mittels welchen eine Anströmung eingestellt werden kann, obgleich die Anströmung dabei meist nicht auf sehr gleichförmige Weise erfolgen kann.

Auch die spezifische Anordnung der Fasern in dem Oxygenatormodul kann die Gasaustauschrate beeinflussen. Es sind Oxygenatormodule bekannt, in welchen die einzelnen Lagen der Hohlfasern übereinander angeordnet sind und die Fasern dabei in einer der Lagen verdreht zu den Fasern in einer benachbarten Lage angeordnet sind. Die Fasern können z.B. um 90 Grad verdreht sein, so dass sich eine kreuzförmige Anordnung ergibt. Mehrere Lagen bilden dann ein kreuzförmiges Faserbündel.

Die Oxygenatormodule werden meist durch Fixieren der Hohlfasern in Vergussmasse hergestellt. Dabei wird die Vergussmasse in eine Form eingebracht, in welcher die Faserlagen angeordnet sind, und durch exzentrische Rotation der Form aufgrund von Zentrifugalkräften an einer Innenmantelfläche der Form angeordnet, und härtet darin aus und bildet eine so genannte Verpottung. Dieser Verfahrensschritt wird dabei für mehrere Seiten des Faserbündels wiederholt, insbesondere viermal, bis das Faserbündel von allen Seiten von der Verpottung eingefasst und fixiert ist. Dadurch kann von der Verpottung eine Kavität gebildet werden, in welcher die Fasern flüssigkeitsdicht gegenüber der Umgebung angeordnet sind und von z.B. Blut umströmt werden können. Danach wird die Verpottung aus der Form entnommen. An der Form sind an allen Seiten so genannte Gießkappen vorgesehen, die nach dem Gießen entfernt werden können, um die Fasern freizulegen. Die Form kann dabei durch die späteren Deckel (oben und unten) eines Oxygenators einerseits und die Gießkappen andererseits gebildet werden. Die Außenmantelflächen der Verpottung können nachbearbeitet werden, um die Enden der Fasern freizulegen und für eine Gasbeschickung zugänglich zu machen.

Nachteilig bei diesen Oxygenatormodulen ist, dass sie nur auf relativ aufwändige Weise hergestellt werden können und dabei häufig auch in der von der Verpottung gebildeten Kavität Kanten, Übergangsbereiche oder Spalte zwischen einzelnen Abschnitten der Verpottung nicht vermieden werden können. Hierdurch entstehen Toträume, in welchen eine Durchströmung nicht auf gleichförmige Weise erfolgen kann, was das Risiko einer Koagulation steigert und die Einsatzdauer des Oxygenatormoduls herabsetzt. Ferner ist nachteilig, dass die erforderliche Menge der Vergussmasse an den einzelnen Seiten des Moduls in bestimmten Fällen unterschiedlich groß zu wählen ist, so dass der entsprechende Arbeitsschritt bei den einzelnen Seiten nicht exakt gleich eingestellt werden kann. Vielmehr sind manuelle Feineinstellungen erforderlich, wodurch weitere Fehlerquellen und Qualitätsschwankungen nicht ausgeschlossen werden können.

Dokument US 6,117,390 ist ein kompakter Blut-Oxygenator zu entnehmen, der ein generell rechteckiges Gehäuse aufweist, in dem in Längsrichtung beabstandete und zwischengelagerte Wärmetauscherleitungsgruppen und Oxygenatorfaserbündel angeordnet sind. Die Leitungen und die Oxygenator-Fasern verlaufen dabei transversal zu einander. Eine ausgehärtete Verpottungsmasse und Flansche an den inneren Oberflächen des Gehäuses definieren dabei hermetisch dichte Strömungswege für das Wärmetransferfluid, Blut und Gasgemische.

Aus der EP 1 864 709 A2 ist ein Membranturm bekannt, der ein Gehäuse aufweist, das einen Stapel von Membranmatten und einen Gehäusedeckel aufweist. Das Gehäuse weist ein geschlossenes Ende und ein offenes Ende auf, wobei das geschlossene Ende eine Auslassöffnung umfasst. Der Gehäusedeckel ist mit dem offenen Ende des Gehäuses verbunden und umfasst eine Einlassöffnung. Ein Stapel von Membranmatten ist in dem Gehäuse im Wesentlichen senkrecht zu dessen Längsachse gestapelt. Jede Membranmappe weist eine Vielzahl von Hohlfaserelementen auf, die über ein Einbettungsmaterial miteinander verbunden sind, wobei das Einbettungsmaterial gleichzeitig das eine Ende des Stapels zu dem geschlossenen Ende des Gehäuses und mit der Kappe verbindet.

Die extrakorporale Lungenunterstützungsvorrichtung gemäß nachveröffentlichter PCT-Anmeldung WO 2014/085620 A1 beschreibt einen Oxygenator mit einem Bluteinlass, der seitlich in der Ebene der Hohlfaserlagen angeordnet ist.

Aufgabe der vorliegenden Erfindung ist es, eine verbesserte Vorrichtung zum Gasaustausch zwischen einem Gas und Blut eines Menschen, Tieres oder separaten Organs, insbesondere für extrakorporale Lungenunterstützungssysteme, bereitzustellen, sowie ein verbessertes Verfahren zum Herstellen einer solchen Vorrichtung bereitzustellen.

Die zuvor genannte Aufgabe wird durch einen Oxygenator gemäß Anspruch 1 gelöst.

Ein erfindungsgemäßer Oxygenator zum Gasaustausch zwischen Blut und einem Gas in einem extrakorporalen Lungenunterstützungssystem weist ein Oxygenatormodul mit mehreren Lagen von semipermeablen vom Gas durchströmbaren Hohlfasern, insbesondere Membranfasern, wobei die Hohlfasern einer der Lagen in einem Verdrehwinkel um eine Mittenlängsachse des Oxygenatormoduls zu den Hohlfasern einer anderen der Lagen ausgerichtet sind, und mit einer Verpottung, welche sich entlang der Mittenlängsachse erstreckt und in welcher die Hohlfasern fixiert sind, wobei die Verpottung eine sich entlang der Mittenlängsachse erstreckende Kavität festlegt, in welcher die Hohlfasern angeordnet sind und welche vom Blut in Richtung der Mittenlängsachse durchströmbar ist, das in dem extrakorporalen Lungenunterstützungssystem so anordenbar ist, dass das Blut an einer Seite in die Kavität eingeleitet werden kann, die Hohlfasern in der Kavität umströmt, und an einer anderen Seite aus der Kavität herausgeleitet werden kann.

Erfindungsgemäß ist vorgesehen, dass die Verpottung eine im Querschnitt im Wesentlichen kreisförmige Innenmantelfläche aufweist, welche die Kavität radial nach außen begrenzt.

Die Innenmantelfläche der Verpottung ist dabei in Bezug auf eine Ebene senkrecht zu der Mittenlängsachse kreisförmig ausgebildet. Durch die im Querschnitt kreisförmige Innenmantelfläche können Ecken und Toträume vermieden werden. Die kreisförmige Innenmantelfläche ist vorzugsweise in sich geschlossen. Sie ist als eine Fläche ausgebildet, die vollständig in einem einzelnen Erstarrungsvorgang gebildet sein kann. Die kreisförmige, bevorzugt zylindrische Innenmantelfläche kann bevorzugt durch Rundverpotten auf einer Zentrifuge erzeugt werden. Unter "Rundverpotten" ist dabei ein Verfahren zu verstehen, bei welchem der Verpottung eine kreisförmige, insbesondere zylindrische (Innen-)Geometrie verliehen wird. Eine Rundverpottung ist demnach eine Verpottung, die innen zur Kavität hin eine möglichst gleichförmig gerundete Geometrie aufweist, wobei ein Krümmungsradius der Verpottung über den Umfang - zumindest in gewissen Grenzen - auch variieren kann. Durch Rundverpottung kann ein zylindrisches Modul oder ein Modul mit im Wesentlichen zylinderischer Kavität bereitgestellt werden, welches bei kurzer Erstreckung in Richtung der Mittenlängsachse im Wesentlichen scheibenförmig ausgebildet ist, also eher flach und breit. Die Außengeometrie der Verpottung kann wahlweise rund oder auch eckig ausgebildet sein, und ist bevorzugt ebenfalls zylindrisch.

Dabei kann mittels winkelig zueinander ausgerichteten Hohlfasern der Gasaustausch verbessert werden, insbesondere da das Blut entlang von Strömungspfaden strömen muss, welche um die einzelnen Hohlfasern herumführen. Die Hohlfasern können sich derart kreuzen, dass in einer Draufsicht auf die Lagen (in Richtung der Mittenlängsachse und senkrecht zu der Ausrichtung der Hohlfasern gesehen) weniger oder keine von einem Bluteinlass zu einem Blutauslass direkt bzw. geradlinig führende Hohlräume bzw. Durchlässe vorliegen. Das strömende Blut kommt dabei mit einer größeren Oberfläche der Hohlfasern in Kontakt. Die kreuzweise oder zumindest um die Mittenlängsachse verdrehte Anordnung der Fasern in Verbindung mit der kreisförmigen Innenmantelfläche, insbesondere einer zylindrischen Kavität, begünstigt ein vollständiges Umströmen der Fasern und damit einen effizienten Gasaustausch. Die Hohlfaserlagen können z.B. als so genannte Membranmatten ausgebildet sein, insbesondere mit einer rechteckigen, beispielsweise quadratischen Grundform. Bevorzugt ist die Grundform der Hohlfaserlagen rechteckig mit unterschiedlicher Seitenlänge. Hierdurch können bei benachbarten, zueinander verdrehten Lagen die einzelnen Lagen jeweils an hervorstehenden Enden in einem bestimmten Winkelbereich um die Mittenlängsachse freigelegt werden, ohne dass die benachbarte Lage in diesem Winkelbereich freigelegt wird. Dies ermöglicht, geschlossene Fasern einzusetzen, welche durch ein Rotationsschneiden einer Außenmantelfläche durch Rotation einer Schneideinrichtung um die Mittenlängsachse an ihren freien Enden geöffnet werden, ohne dass die Gefahr besteht, dass sie an ihren Längsseiten aufgeschnitten werden. Weiter bevorzugt ist die Seite der rechteckigen Grundform länger, parallel zu welcher die Hohlfasern angeordnet sind. Hierdurch kann auf einfache Weise ein Zugang zu den innerhalb der Verpottung fixierten und zumindest teilweise von der Verpottungsmasse umschlossenen Fasern geschaffen werden.

Als Mittenlängsachse ist dabei bevorzugt eine Achse zu verstehen, um welche die Verpottung angeordnet wird, und entlang welcher das Blut von einem Einlass durch die Kavität zu einem Auslass strömt.

Die semipermeablen Hohlfasern können mit einem sauerstoffhaltigen Gas beschickt werden und sind sauerstoffdurchlässig. Als semipermeable ist dabei eine Faser aufzufassen, welche gasdurchlässig (z.B. für Gasdiffusion durchlässig) ist, jedoch flüssigkeitsdicht, insbesondere blutdicht ist.

Als Verpottungsmaterial kann z.B. Polyurethan oder Silikon eingesetzt werden.

Als eine Membranfaser ist dabei bevorzugt eine Hohlfaser zu verstehen, welche als semipermeable dünne Kapillaren mit poröser Wandung ausgebildet sind. Sie weisen bevorzugt eine zylindrisch ausgebildete Form mit einem inneren zylindrischen Hohlraum auf. Im Falle von mikroporösen Membranen (insbesondere für Kurzzeitanwendungen) kann z.B. verstrecktes Polypropylen (PP) eingesetzt werden. Im Falle von plasmadichten Membranen für die Langzeitanwendung kann z.B. Polymethylpenten (PMP) mit plasmadichter Haut auf der (blutseitigen) Oberfläche der Fasern eingesetzt werden. Bevorzugt werden PMP-basierte Fasern eingesetzt.

Als ein Oxygenatormodul ist dabei bevorzugt eine Vorrichtung zu verstehen, welche den Austausch von Gasbestandteilen zwischen Blut und einem innerhalb des Blutes in einem Leitungssystem, insbesondere Hohlfasermembranen, geförderten Gases ermöglicht, insbesondere Sauerstoff und Kohlendioxid. Ein Oxygenatormodul weist Faserlagen oder Fasermatten auf, welche in dem Oxygenatormodul z.B. aufeinandergeschichtet, gefaltet und/oder gelegt angeordnet sind und ein gelegtes Oxygenatormodul bilden. Als ein gelegtes Oxygenatormodul ist dabei bevorzugt ein Oxygenatormodul zu verstehen, bei welchem einzelne Lagen von Hohlfasern aufeinander angeordnet sind und ein Bündel bilden. Das Oxygenatormodul weist ferner eine Verpottung auf, in welche die Fasern eingebettet sind. Wahlweise kann das Oxygenatormodul auch einen oder mehrere Deckel zum Abdichten der von der Verpottung gebildeten Kavität aufweisen.

Bevorzugt ist die Innenmantelfläche der Kavität bzw. der Verpottung entlang der Mittenlängsachse in einem konstanten radialen Abstand zu der Mittenlängsachse angeordnet, d.h., die Innenmantelfläche ist zylindrisch ausgebildet, so dass der Kavität durch die Verpottung eine zylindrische Geometrie vorgegeben ist. Durch die zylindrische Innenmantelfläche der Rundverpottung kann vermieden werden, dass sich an Ecken oder in Nischen Toträume bilden, die wenig oder gar nicht von Blut durchströmt werden. Durch eine zylindrische Kavität kann eine gleichförmige Durchströmung sichergestellt werden. Nicht oder zu wenig durchströmte Abschnitte (so genannte Totwassergebiete) können vermieden werden, wodurch die Gefahr von Koagulation reduziert und die Einsatzdauer des Oxygenatormoduls verlängert wird.

Die Lagen sind bevorzugt in Ebenen angeordnet, die sich in einem Winkel von 45 bis 90 Grad, insbesondere zumindest annähernd orthogonal zu der Mittenlängsachse erstrecken. Hierdurch können sie zum einen gut in der Verpottungsmasse fixiert werden, zum anderen kann eine große Anzahl von Fasern in einer Kavität mit einem vorgegebenen Innenvolumen angeordnet werden. Bevorzugt sind die Lagen in parallelen Ebenen zueinander angeordnet. Die Hohlfasern einer Lage können ferner zumindest annähernd orthogonal zu den Hohlfasern einer benachbarten Lage angeordnet sein, so dass ein kreuzförmiges Faserbündel gebildet ist. Diese (insbesondere kreuzförmige) Anordnung liefert gute strömungstechnische Eigenschaften und kann zudem auf einfache Weise hergestellt werden. Gemäß einer bevorzugten Variante sind die Fasern einer Lage in einem Winkel von zumindest annähernd 45 oder 60 Grad zu den Fasern einer benachbarten Lage angeordnet, wobei bevorzugt drei oder vier aufeinanderfolgende Lagen jeweils um einen Winkel von 45 oder 60 Grad um die Mittenlängsachse verdreht sind.

Weiter bevorzugt sind die Lagen in radialer Richtung versetzt zueinander angeordneten, so dass in einer Strömungsrichtung gesehen das Blut direkt auf Hohlfasern trifft, um die es herum strömen muss. Eine solche Anordnung kann als statischer Mischer bezeichnet werden. Hierdurch kann vermieden werden, dass der Blutstrom hauptsächlich entlang von kanalartigen Hohlräumen in der Kavität erfolgt und die Faseroberfläche nicht vollständig umströmt. Die radial versetzte Anordnung kann in Verbindung mit der kreuzförmigen oder um 45 oder 60 Grad verdrehten winkeligen Anordnung vorgesehen sein.

Das Gas ist dabei insbesondere Sauerstoff oder ein sauerstoffhaltiges Gas oder auch Raumluft. Die Hohlfasern können vom Typ Membranfasern für einen Oxygenator sein, jedoch sind auch andere Fasern verwendbar, insbesondere wenn das Modul nicht für einen Oxygenator vorgesehen ist, sondern für andere aber ähnliche Anwendungen, zum Beispiel für einen Wärmetauscher, einen Hämofilter, ein Dialysegerät, einen arteriellen Filter, oder für kombinierte Geräte, also Geräte mit z.B. sowohl Gasaustausch- als auch Wärmetauscherfunktion.

Erfindungsgemäß sind die Lagen jeweils teilweise überlappend zueinander angeordnet, wobei mindestens ein freies Ende der Lagen jeweils in einem nicht überlappenden Bereich von einer unmittelbar benachbarten Lage hervorsteht. Hierzu weisen die Lagen bevorzugt eine rechteckige Grundform mit unterschiedlicher Seitenlänge auf. Bevorzugt stehen beide freien Enden der Lagen jeweils in einem nicht überlappenden Bereich von einer benachbarten Lage hervor. Weiter bevorzugt sind die teilweise überlappenden Lagen bei kreuzförmig oder um 45 oder 60 Grad verdreht zueinander angeordneten Faserbündeln oder -lagen vorgesehen. Durch eine nur teilweise überlappende Anordnung können die freien Enden der Fasern besonders nah an einer Innenwandung einer vorzugsweise im Wesentlichen zylindrischen Form angeordnet werden, in welche Verpottungsmasse eingebracht wird. Hierdurch kann zum einen Verpottungsmasse eingespart werden, zum anderen kann auf einfachere Weise ein Verfahrensschritt zum Freilegen der Faserenden eingespart oder zumindest auf einfache oder schnelle Weise durchgeführt werden. Gemäß einer Variante können die Lagen auch eine kreisförmige Geometrie aufweisen, insbesondere in Bezug auf den Bereich, welcher von der Verpottungsmasse umgrenzt ist. Mit anderen Worten kann jede Lage eine vollständige/komplette Querschnittsfläche der Kavität bilden. Der Vorteil von teilweise überlappenden Lagen kann am Beispiel von jeweils um 45 oder 60 Grad zueinander verdreht angeordneten Lagen beschrieben werden, wobei jede Lage eine rechteckige Grundform mit unterschiedlicher Seitenlänge aufweist. Die Lagen sind bevorzugt jeweils deckungsgleich. Jeweils drei oder vier Lagen weisen zusammen drei oder vier unterschiedliche Bereiche oder Abschnitte unterschiedlicher Faserdichte auf. In einem Kernbereich überlappen sich alle drei oder vier Lagen. Der Kernbereich, welcher die größte Faserdichte in Bezug auf das eingenommene Volumen der Kavität aufweist, weist dabei bevorzugt eine hexagonale Grundform auf. In einem jeweiligen hervorstehenden Bereich oder Abschnitt überlappt keine der drei oder vier Lagen die anderen Lagen. Insgesamt werden sechs oder acht solcher nicht überlappenden, freiliegenden Abschnitte gebildet. Die nicht überlappenden, freiliegenden Abschnitte weisen bei einer 60 Grad-Anordnung z.B. jeweils eine dreiecksförmige Geometrie mit sich nach radial außen hin daran anschließendem rechteckigem Abschnitt auf. In diesen Abschnitten ist die Faserdichte am geringsten. Ferner werden auch teilweise überlappende Abschnitte gebildet, in welchen zwei der drei Lagen oder drei der vier Lagen einander überlappen. Die teilweise überlappenden Abschnitte weisen bei einer 60 Grad-Anordnung z.B. jeweils eine dreiecksförmige Geometrie auf.

Gemäß einer bevorzugten Ausführungsform weist die Verpottung eine insbesondere zylindrische Außenmantelfläche auf, aus welcher die Hohlfasern mit mindestens einem freien Ende radial hervorstehen. Hierdurch kann das Oxygenatormodul zum einen mit wenigen Arbeitsschritten hergestellt werden, zum anderen können die Faserenden allesamt dieselbe Geometrie aufweisen. Ein Abtrennen, insbesondere Schneiden der (freien Enden der) Hohlfasern ist nicht notwendigerweise erforderlich. Die Faserenden werden z.B. nicht durch ein Schneiden mit einem Grat versehen oder versprödet oder gebrochen oder elliptisch. Die Faserenden können dabei z.B. auch mit einer bestimmten Geometrie ausgebildet sein, z.B. leicht konisch oder mit einer Fase, um die Einströmung von Gas besonders gleichförmig zu gestalten. Für den Fall dass die Faserenden noch nicht offen sind, kann ein Schneiden der Faserenden auf einfachere Weise erfolgen, insbesondere da die Faserenden frei vorliegen und nicht in der Vergussmasse eingegossen sind.

Bevorzugt ist die Außenmantelfläche zylindrisch ausgebildet. Als eine zylindrische Außenmantelfläche ist dabei eine Mantelfläche zu verstehen, welche im Querschnitt eine zumindest annähernd kreisförmige Geometrie aufweist. Weiter bevorzugt ist die Verpottung rohrförmig ausgebildet. Eine rohrförmige Verpottung ist dabei durch eine vollständig zylindrische Innenmantelfläche und eine vollständig zylindrische Außenmantelfläche gebildet. Bei einer zylindrischen Außenmantelfläche können die Faserenden auf einfache Weise zugänglich gehalten werden oder zugänglich gemacht werden, insbesondere indem die Außenmantelfläche mit einer relativ zum Oxygenatormodul rotierenden Schneideinrichtung bearbeitet wird.

Mindestens zwei der Lagen können aus einer gefalteten Fasermatte gebildet sein, wobei die Hohlfasern der gefalteten Fasermatte in mehreren der Lagen angeordnet sein können. Mit anderen Worten ist jeweils eine (lange) Hohlfaser in mindestens zwei unterschiedlichen Lagen angeordnet. Eine gefaltete Fasermatte kann z.B. durch eine Girlandenfaltung von zwei schmalen Matten aus langen Hohlfasern gebildet werden. Alternativ kann eine gefaltete Fasermatte z.B. auch durch eine Faltung von zwei Matten mit sich regelmäßig wiederholenden Faserleerstellen, also zu einzelnen Paketen zusammengefassten Hohlfasern, gebildet werden. Ein Vorteil bei gefalteten Lagen besteht auch darin, dass der Schritt einer Vereinzelung in einzelne Lagen entfallen kann, denn dieser Schritt ist je nach Ausgestaltung der Faserenden entbehrlich oder kann in Verbindung mit einem Öffnen bzw. Schneiden der Faserenden erfolgen. Die lange Hohlfaser kann wahlweise durch Schneiden nach einem Verpotten wieder unterteilt werden, so dass in jeder Lage die Hohlfasern unabhängig voneinander durchströmbar sind.

Gemäß einer Ausführungsform, welche mit einer der zuvor beschriebenen Ausführungsformen kombiniert werden kann, weisen die Hohlfasern einer jeweiligen Lage zwei freie Enden auf und stehen mit beiden freien Enden aus der Verpottung, insbesondere der Außenmantelfläche, hervor. Die Fasern sind dabei bevorzugt an ihren beiden freien Enden offen, oder wurden insbesondere durch Schneiden geöffnet. Hierdurch kann das Oxygenatormodul auf kostengünstige Weise und/oder mit wenigen Verfahrensschritten hergestellt werden. Ein Öffnen der Hohlfasern, insbesondere durch Schneiden, ist nicht mehr erforderlich. Als offene Hohlfasern sind dabei Hohlfasern zu verstehen, welche im Rohzustand als Halbzeug bereits in unverschlossener Form vorliegen, also nicht mehr durch ein Aufschneiden der freien Enden geöffnet werden müssen. Wird eine Hohlfaserlage hergestellt, indem eine Endlosfaser mäanderförmig gelegt und mit Kettfäden fixiert wird, so liegen üblicherweise geschlossene Enden vor, welche aufgeschnitten werden.

Gemäß einer Ausführungsform, welche mit einer der zuvor beschriebenen Ausführungsformen kombiniert werden kann, sind mindestens zwei der Lagen aus einer gefalteten Hohlfasermatte gebildet, wobei die Hohlfasern in der Hohlfasermatte als Hohlfaserpakete beabstandet zueinander angeordnet sind und mittels Kettfäden miteinander verbunden sind. Mit anderen Worten sind die (kurzen) Hohlfasern der Fasermatte jeweils zu Paketen zusammengefasst, welche eine der Lagen bilden, und jede Lage ist unabhängig von den anderen Lagen. Hierdurch kann auf einfache Weise ein Faserbündel mit nur teilweise überlappenden Lagen bereitgestellt werden.

Gemäß einer bevorzugten Ausführungsform, welche mit einer der zuvor beschriebenen Ausführungsformen kombiniert werden kann, weist das Oxygenatormodul eine Außengeometrie mit mehr als vier Ecken auf, insbesondere eine hexagonale oder eine oktagonale Außengeometrie. Durch diese Geometrie kann die von den Fasern bereitgestellte Austausch-Oberfläche besonders gut ausgenutzt werden, insbesondere in Verbindung mit einer zylindrischen Kavität. Die Außengeometrie kann auf zweckmäßige Weise in Verbindung mit einer vorteilhaften Anordnung der Lagen in einem Winkel zueinander vorgesehen sein. Bevorzugt wird die Außengeometrie sowohl durch die Verpottung als auch durch mindestens einen Deckel gebildet.

Gemäß einer bevorzugten Ausführungsform, welche mit einer der zuvor beschriebenen Ausführungsformen kombiniert werden kann, sind mindestens zwei, bevorzugt mindestens drei, der Lagen in einem Winkel ungleich Null und kleiner als 90 Grad verdreht zueinander, insbesondere verdreht um die Mittenlängsachse, angeordnet, bevorzugt in einem Winkel von zumindest annähernd 45 oder 60 Grad verdreht zueinander. Diese Anordnung kann eine besonders effektive Ausnutzung der Faseroberfläche als Austauschfläche sicherstellen. Beim Stapeln der Lagen in einem Winkel von 90 Grad in einer zylindrischen Kavität wäre ein Großteil der Fasern von Verpottungsmaterial umgeben. Hierdurch ist prozentual ein kleinerer Anteil der Fasern in Kontakt mit Blut. Im Vergleich zu einer solchen kreuzförmigen (orthogonalen) Anordnung kann bei einer 45 Grad- oder 60 Grad-Anordnung die Oberfläche der Fasern oder der zur Verfügung stehende Raum bzw. das Volumen der Kavität besonders effektiv ausgenutzt werden, insbesondere in Verbindung mit einer zylindrischen Kavität. Es ergeben sich im Vergleich zu einer kreuzförmigen (90 Grad) Anordnung noch weniger Toträume oder Freiräume, in welchen nur ein reduzierter Austausch erfolgen kann. Der Verdrehwinkel ist bevorzugt deutlich kleiner als 90 Grad und beträgt bevorzugt maximal 60 Grad. Der Verdrehwinkel muss jedoch nicht notwendigerweise genau 45 Grad oder 60 Grad betragen, sondern kann auch größer als 60 Grad oder kleiner als 45 Grad sein.

Die um 45 oder 60 Grad verdrehte Anordnung kann sicherstellen, dass die Fasern in drei oder vier unterschiedlichen Orientierungen angeordnet sind, wobei in jeder der drei oder vier unterschiedlich ausgerichteten Faserlagen wahlweise ein spezifisches Fluid geführt werden kann. Beispielsweise kann eine der drei oder vier Faserlagen mit Wasser durchströmt werden, insbesondere in einer Funktion als Wärmetauscher. Dabei können drei oder vier unterschiedliche Strömungsrichtungen vorgegeben werden. Gemäß einer Variante handelt es sich bei den Fasern bzw. dem Fasermaterial der jeweiligen Lage jeweils um ein spezifisches Fasermaterial, welches z-B- in Hinblick auf das zu führende Fluid/Medium optimiert ist.

Bei der um 45 oder 60 Grad verdrehten Anordnung können Randbereiche mit im Vergleich zu einem Kernbereich verringerter Faserdichte bereitgestellt werden, wobei in den Randbereichen eine Vorzugsströmung eingestellt werden kann, was insbesondere bei einer großen Querschnittsgeometrie Vorteile für den Austausch liefert. Mit anderen Worten: Gewöhnlich ist die Strömungsgeschwindigkeit dort am geringsten, wo der Abstand zu Strömungseinlass/-auslass am größten ist. Eine geringe Strömungsgeschwindigkeit bedeutet häufig auch ein größeres Koagulationsrisiko, so dass Variationen in der Strömungsgeschwindigkeit möglichst vermieden werden sollten. Bei zentrischer Anströmung ist die Strömungsgeschwindigkeit insbesondere im Randbereich der Kavität geringer als im Zentrum. Je größer der Durchmesser der Kavität, desto größer die Variation der Strömungsgeschwindigkeit zwischen dem Zentrum und dem Rand der Kavität. Durch die verminderte Faserdichte im Randbereich kann der Strömungswiderstand im Randbereich gesenkt werden, z.B. auf 1/3 oder 2/3 des Strömungswiderstands bei maximaler Faserdichte, wodurch im Randbereich eine vergleichsweise größere Strömungsgeschwindigkeit eingestellt werden kann. Die Strömung fließt bevorzugt durch diesen Randbereich mit geringerem Widerstand (Vorzugsströmung). Je nach Ausprägung des Verdrehwinkels der Faserlagen können dabei Strömungsunterschiede zwischen dem Zentrum und dem Randbereich ausgeglichen werden, so dass ein homogenes Strömungsfeld realisiert werden kann und die Verweildauern bzw. Kontaktzeiten angeglichen werden können.

Bei der um 45 oder 60 Grad verdrehten Anordnung kann zudem auch Fasermaterial eingespart werden. Auch kann ein Gehäuse besonders kompakt ausgeführt werden. Bevorzugt sind alle Hohlfaserlagen verdreht zueinander angeordnet, insbesondere benachbarte, direkt aufeinanderliegende Hohlfaserlagen jeweils in einem Verdrehwinkel von zumindest annähernd 45 Grad oder 60 Grad.

Bevorzugt weist das Oxygenatormodul eine hexagonale Außengeometrie oder Umfangsgeometrie auf, wobei jede Seite oder Kante der Umfangsgeometrie bevorzugt durch freiliegende Faserenden einer jeweiligen Faserlage definiert ist.

Gemäß einer Variante können unterschiedliche Verdrehwinkel miteinander kombiniert werden, insbesondere auch in einem einzigen Oxygenatormodul.

Ein erfindungsgemäßer Oxygenator ist mit mindestens einem Oxygenatormodul ausgeführt. Es können wahlweise mehrere Oxygenatormodule in Reihe hintereinander angeordnet sein.

Ein Oxygenatormodul, vorzugsweise ein Oxygenatormodul mit den zuvor beschriebenen Eigenschaften, ist in einem erfindungsgemäßen Oxygenator zum Gasaustausch zwischen Blut und einem Gas in einem extrakorporalen Lungenunterstützungssystem vorgesehen, mit einem Bluteinlass und einem Blutauslass, jeweils gekoppelt (in Kommunikation) mit einer blutdurchströmbaren Kavität des Oxygenatormoduls, und mit einem Gehäuse zur Aufnahme des Oxygenatormoduls, wobei das Gehäuse einen Gaseinlass und einen Gasauslass aufweist, welche jeweils mit Hohlfasern des Oxygenatormoduls gekoppelt sind, d.h., der Gaseinlass und der Gasauslass stehen in strömungstechnischer Verbindung mit den Hohlfasern.

Der erfindungsgemäße Oxygenator weist eine Verteilereinrichtung auf, welche in Bezug auf eine Mittenlängsachse des Oxygenatormoduls stromauf von dem mindestens einen Oxygenatormodul und stromab von dem Bluteinlass angeordnet ist, wobei der Oxygenator für eine zentrische Anströmung des Oxygenatormoduls eingerichtet ist. Hierdurch kann der Blutstrom homogener auf das Hohlfaserbündel verteilt werden, und eine homogene Durchströmung kann auf effektivere Weise sichergestellt werden. Bei einem rundverpotteten Oxygenator mit zylindrischer Kavität ist dabei der radiale Abstand des Bluteinlasses zum Randbereich in allen radialen Richtungen gleich.

Gemäß einer Ausführungsform ist der Bluteinlass und/oder der Blutauslass zentrisch in Bezug auf das Oxygenatormodul angeordnet, insbesondere in Bezug auf die Kavität. Hierdurch kann eine homogene Durchströmung und eine homogene Aufweitung des Stroms sichergestellt werden. Gemäß einer Variante weist die Verteilereinrichtung den Bluteinlass auf und ist derart am Oxygenatormodul angeordnet, dass das Oxygenatormodul zentrisch anströmbar ist.

Die Verteilereinrichtung des erfindungsgemäßen Oxygenators weist einen Drallverteiler auf und ist dazu ausgebildet, einen Blutstrom mit einem Drall in einem Strömungswinkel zu der Mittenlängsachse in die Kavität zu lenken. Hierdurch kann die Blutströmung so in die Kavität geleitet werden, dass die Gefahr von Toträumen vermindert ist. Ferner kann der Gasaustausch beispielsweise im Bereich von 5 bis 12 Prozent in Bezug auf vorbekannte Oxygenatoren verbessert werden. Durch eine homogenere Durchströmung bzw. eine gleichförmigere Verteilung der Strömungsgeschwindigkeiten über die durchströmte Fläche kann die Verweildauer einheitlicher eingestellt werden.

Der Drallverteiler ist dazu ausgebildet, den Blutstrom auf einen Strömungspfad in die Kavität zu lenken, welcher in einem Strömungswinkel zu der Mittenlängsachse ausgerichtet ist. Der Strömungspfad führt dabei nicht notwendigerweise vom Bluteinlass direkt zum Blutauslass, sondern kann länger als der direkte Weg sein. Hierdurch kann der Gasaustausch verbessert werden. Der Drallverteiler ist in Reihe zu dem Oxygenatormodul angeordnet. Bevorzugt wird der Drallverteiler zentral angeströmt und ist symmetrisch in radialer Richtung ausgebildet, insbesondere mit einem gleichen radialen Abstand zu einer Innenmantelfläche der Verpottung bzw. Kavität zu allen Seiten.

Bevorzugt liegt der Strömungswinkel im Bereich von 30 bis 90 Grad, weiter bevorzugt 45 bis 85 Grad, besonders bevorzugt 65 bis 80 Grad. Hierdurch kann eine verbesserte, vollständigere Umströmung der Fasern erzielt werden, insbesondere bei einer zylindrischen Innenmantelfläche einer Kavität. Auch kann sichergestellt werden, dass alle Bereiche der Kavität durchströmt werden. Dabei kann z.B. verhindert werden, dass sich Blutströmung im Wesentlichen nur im Zentrum der Kavität ausbildet. Bevorzugt wird die Stärke der Umlenkung in Abhängigkeit der radialen Erstreckung der Kavität gewählt, wobei die Umlenkung stärker ist, wenn die radiale Erstreckung größer ist.

Als Drallverteiler ist dabei bevorzugt eine bezüglich des Oxygenatorgehäuses statisch angeordnete Vorrichtung zum Umlenken des Blutstroms aufzufassen, wobei die Umlenkung je nach Anordnung der Fasern mit bis zu 90 Grad erfolgen kann. Hierdurch kann das Blut zum einen in eine Rotation versetzt werden, zum anderen auch seitlich, also in einem Winkel in Bezug auf die Mittenlängsachse, durch den Oxygenator geleitet werden und über einen längeren Weg an den Hohlfasern entlang strömen. Der Drallverteiler ermöglicht ein flächiges Aufspreizen des Blutstromes. Insbesondere bei kreuzförmig oder um 45 oder 60 Grad verdreht zueinander angeordneten Fasern bzw. Lagen und einer zylindrischen Innenmantelfläche ist es vorteilhaft, den Blutstrom umzuleiten und zumindest annähernd in Umfangsrichtung entlang der Innenmantelfläche zu leiten, da hierdurch ein größerer Längenanteil der Hohlfasern in Erstreckungsrichtung der Fasern umströmt werden kann.

Der Drallverteiler weist bevorzugt eine zylindrische Außenmantelfläche auf. Hierdurch kann er auf einfache Weise mit einem Oxygenatormodul mit zylindrischer Innenmantelfläche und wahlweise auch zylindrischer Außenmantelfläche gekoppelt bzw. verbunden werden.

Der Gasein- und Gasauslass sind bevorzugt derart angeordnet, dass ein Gasstrom frontal auf freie Enden der Hohlfasern gerichtet werden kann. Dabei kann Gas durch Unter- und/oder Überdruck eingebracht werden, also auf saugende und/oder blasende Weise. Die Verpottung ist dabei in dem Gehäuse an einer Innenmantelfläche des Gehäuses lagerbar und/oder mittels eines oder mehrerer Deckel in dem Gehäuse lagerbar und/oder mit dem Gehäuse verklebt. Die Innenmantelfläche des Gehäuses kann zumindest teilweise zylindrisch ausgebildet sein, insbesondere mit einem zylindrischen radial nach innen hervorstehenden Teil, an welchem die Verpottung lagerbar ist, ohne dass die Faserenden mit der Innenmantelfläche in Berührung gelangen.

Gemäß einer Ausführungsform, welche mit einer der zuvor beschriebenen Ausführungsformen kombiniert werden kann, weist der Drallverteiler eine im Querschnitt kreisförmige Innenmantelfläche sowie innenliegende Drallelemente auf, welche zu einem Mittelpunkt des Drallverteilers hin ineinander zusammenlaufen. Hierdurch kann eine Umlenkung des Blutstroms auf besonders effektive Weise und mit großen Umlenkwinkeln erfolgen, so dass der Blutstrom seitlich mit einem möglichst großen Strömungswinkel im Bereich von 90 Grad in die Kavität geleitet werden kann.

Gemäß einer Ausführungsform, welche mit einer der zuvor beschriebenen Ausführungsformen kombiniert werden kann, weist der Drallverteiler Drallelemente in Form von Flügeln auf, insbesondere vier bis sechs Flügel. Hierdurch kann der Blutstrom über den gesamten Querschnitt des Drallverteilers gleichförmig umgelenkt werden. Die Anzahl der Flügel kann dabei in Abhängigkeit der Geometrie bzw. Abmessung der Kavität gewählt werden, wobei die Flügel zentral aneinander grenzen und eine Prallfläche bilden.

Gemäß einer Ausführungsform, welche mit einer der zuvor beschriebenen Ausführungsformen kombiniert werden kann, weist der Oxygenator stromauf von der Verteilereinrichtung einen Deckel auf, welcher in Richtung der Mittenlängsachse eine pyramidal oder konisch zulaufende Geometrie hat. Hierdurch kann der Blutstrom von einem Bluteinlass bis zum Oxygenatormodul gleichförmig aufgefächert bzw. aufgeweitet und auf den gesamten Querschnitt verteilt werden.

Gemäß einer bevorzugten Ausführungsform weist der Oxygenator mindestens einen Deckel auf, welcher mittels einer/der Verpottung des Oxygenatormodul fixiert ist. Hierdurch kann sichergestellt werden, dass das Oxygenatormodul, insbesondere eine Außenmantelfläche der Verpottung oder Faserenden, auf besonders praktikable Weise nachbearbeitet werden kann. Insbesondere ergibt sich auch der Vorteil, dass die Fasern bei der Bearbeitung nicht frei liegen und nicht beschädigt oder verschmutzt werden können. Dabei weist der Deckel bevorzugt eine hexagonale Außengeometrie auf. Durch diese Geometrie kann der (jeweilige) Deckel zusammen mit den Lagen auf praktikable Weise in einer Vergussform angeordnet werden, wobei der/die Deckel zusammen mit den Lagen mittels Verpottungsmasse fixiert werden können, insbesondere in Verbindung mit einer zylindrischen Kavität und einer 45 oder 60 Grad-Anordnung der Lagen. Der Deckel weist bevorzugt eine Außengeometrie auf, welche geometrisch korrespondierend zur Außengeometrie der Verpottung ausgebildet ist.

Gemäß einer Ausführungsform, welche mit einer der zuvor beschriebenen Ausführungsformen kombiniert werden kann, weist die Verteilereinrichtung weiterhin einen Aspektverteiler oder einen Tangentialverteiler oder einen Verteiler jeweils für eine Aufweitung des Blutstroms auf. Die Aufweitung erfolgt in radialer Richtung senkrecht zu der Mittenlängsachse. Hierdurch kann der Blutstrom flächig auf den Drallverteiler verteilt werden, so dass der Drallverteiler den Blutstrom effektiver umlenken kann. Bevorzugt wird der Aspektverteiler jedoch in Verbindung mit dem Drallverteiler eingesetzt, und insbesondere stromauf vom Drallverteiler angeordnet. Der Aspektverteiler kann auch selbst ein oder mehrere Drallelemente aufweisen und dadurch zu einem Drallverteiler weitergebildet sein. Der Tangentialverteiler weist keine Drallelemente auf und liefert den Vorteil, dass bei einer seitlichen Einströmung und Rotation des Blutstroms Luftblasen vermieden werden, die nicht entweichen können, sondern in der Mitte des Tangentialverteilers in axialer Richtung entweichen können. Ein weiterer Vorteil ist auch die vergleichsweise geringe Bauhöhe des Tangentialverteilers.

Der Aspektverteiler weist eine zentrische Öffnung bzw. Blende auf, welche im Verhältnis zu seinen radialen Abmessungen klein ist. Die radiale Abmessung der Blende liegt bevorzugt bei einem Verhältnis von 1:5, weiter bevorzugt 1:10 zu der absoluten radialen Abmessung des Aspektverteilers. Der Tangentialverteiler weist eine zentrische Öffnung bzw. Blende auf, welche im Verhältnis zu seinen radialen Abmessungen klein ist. Die radiale Abmessung der Blende liegt bevorzugt bei einem Verhältnis von mehr als 1:7, weiter bevorzugt 1:12 oder 1:15 zu der absoluten radialen Abmessung einer Innenmantelfläche des Tangentialverteilers.

Gemäß einer Ausführungsform, welche mit einer der zuvor beschriebenen Ausführungsformen kombiniert werden kann, weist die Verteilereinrichtung ein Verteilelement mit einer Vielzahl von Durchlässen auf, insbesondere eine kreisrunde Blutverteilerplatte, welche geometrisch korrespondierend zur Kavität ausgebildet ist. Ein solches Verteilelement kann platzsparend zwischen weiteren Komponenten des Oxygenators angeordnet werden und den Blutstrom durch eine einfache Maßnahme auf effiziente Weise über den gesamten Querschnitt verteilen.

Auch offenbart wird ein Verfahren zum Herstellen eines Moduls für eine Mehrfluidfaservorrichtung, insbesondere eines Oxygenatormoduls für einen Oxygenator eines extrakorporalen Lungenunterstützungssystems, mit den Schritten:
a) Anordnen einer Mehrzahl von Hohlfaserlagen innerhalb einer (Verguss-)Form, wobei die Hohlfasern einer der Lagen in einem Verdrehwinkel um eine Mittenlängsachse des Oxygenatormoduls zu den Hohlfasern einer anderen der Lagen ausgerichtet werden;
b) Anordnen der Form in Bezug auf eine Drehachse einer Zentrifuge;
c) Zuführen von Verpottungsmasse in die Form;
d) Drehen der Form um die Drehachse zum Ausüben einer Zentrifugalkraft auf die Verpottungsmasse zum Anordnen der Verpottungsmasse radial außen in der Form, insbesondere zeitgleich mit Schritt c);
e) Aushärten der Verpottungsmasse zum Fixieren der Hohlfaserlagen, insbesondere bei fortwährendem Drehen;
f) Entnehmen der Verpottungsmasse zusammen mit den Hohlfaserlagen aus der Form oder zumindest aus einem Teil der Form.

Durch ein solches Verfahren kann ein Oxygenatormodul für ein extrakorporales Lungenunterstützungssystem bereitgestellt werden.

Offenbart wird also, dass in Schritt b) die Form derart um die Drehachse angeordnet wird, dass die Drehachse innerhalb der Form liegt, wobei in Schritt d) eine Kavität mit einer im Wesentlichen kreisförmigen Innenmantelfläche der Verpottungsmasse gebildet wird. Hierdurch kann eine gleichmäßige Rundverpottung ohne Spalten, kantige Übergänge oder sonstige Unstetigkeiten, die Toträume begünstigen, bereitgestellt werden. Insbesondere kann ein durchströmbarer Bereich mit kreisrunder, zylindrischer Querschnittsgeometrie bereitgestellt werden, welcher gleichförmig von Blut durchströmt werden kann.

Gemäß einem Aspekt werden die Lagen in Schritt d) derart in der Verpottungsmasse eingebettet, dass sie teilweise überlappend zueinander angeordnet sind. Durch die (nur) teilweise überlappende Anordnung der Lagen relativ zueinander kann sichergestellt werden, dass die Lagen jeweils nahe im Bereich der Faserenden eingebettet werden können. Die Faserenden ragen vom überlappenden Kernbereich hervor. Auch bei einer geradlinigen Anordnung der Faserenden kann eine jeweilige Lage mit in radialer Richtung gesehen vergleichsweise wenig Verpottungsmaterial eingebettet werden. Ferner kann eine jeweilige Lage auch auf sicherere Weise in der Verpottungsmasse eingebettet werden, weil mehr Verpottungsmasse die Lage oberhalb und unterhalb der Lage umgeben kann als bei einer Faserdichte entsprechend der Faserdichte im Kernbereich.

Das Verpottungsmaterial wird dabei in eine Vergussform eingebracht. Die Vergussform kann sich insbesondere durch folgende Merkmale auszeichnen, sei es allein oder in Kombination miteinander:
- symmetrischer Aufbau, insbesondere zur Vermeidung von Unwuchten;
- zweiteilig, insbesondere zum Einbringen oder Entfernen von Komponenten wie z.B. Deckel oder Faserlagen;
- flüssigkeitsdicht;
- Innengeometrie mit mehr als vier Ecken, bevorzugt hexagonale Innengeometrie;
- Entformungsschrägen;
- Öffnungen zum Ausstoßen des Moduls.

Dabei kann die Vergussform einen mitrotierenden Verteilertrichter mit bevorzugt vier bis sechs Abgängen aufweisen.

Die Drehachse ist dabei in einem radialen Abstand zu der Innenmantelfläche der Form angeordnet. Der Abstand der Drehachse zu der Form ist bevorzugt größer als die zu erzielende Mindestdicke der Verpottung in radialer Richtung. Bevorzugt wird in Schritt b) die Form zentrisch um die Drehachse angeordnet. Hierdurch kann eine Verpottung mit einheitlicher Wandstärke bereitgestellt werden. Im Gegensatz zu einem exzentrischen Verpotten kann dabei auch mindestens ein Arbeitsschritt eingespart werden, denn die Verpottung kann vollständig erfolgen, ohne dass die Form neu auf der Zentrifuge positioniert (umpositioniert) werden muss. Das Verpotten kann vollständig in Bezug auf alle Seiten der Hohlfaserlagen bzw. des Faserbündels in einem Verfahrensschritt erfolgen. Hierdurch kann das Faserbündel auch genauer innerhalb der Verpottung fixiert werden, denn es kann eine Unterbrechung des Verfahrens zu einem Zeitpunkt vermieden werden, zu welchem das Faserbündel nur an einer Seite in Verpottungsmasse fixiert wäre. Ferner kann der Innendurchmesser der Kavität auf einfache Weise eingestellt werden, denn es ist dazu lediglich erforderlich, eine bestimmte Menge für die Verpottungsmasse zu wählen. Dabei ergibt sich auch der vorteilhafte Effekt, dass die einzelnen Hohlfasern nicht gestaucht oder durchgebogen werden, sondern allenfalls aufgrund der Zentrifugalkraft gestreckt werden, wodurch die Lage der Hohlfasern zueinander eindeutiger bzw. genauer eingestellt werden kann. Auch dies stellt eine homogenere Umströmung der Hohlfasern sicher.

Die Innenmantelfläche ist in Bezug auf eine Ebene senkrecht zur Mittenlängsachse zumindest annähernd kreisförmig ausgebildet, so dass eine gleichförmig durchströmbare Kavität bereitgestellt werden kann. Die Kavität weist keine Hinterschneidungen, Kanten oder sonstigen unstetigen Übergänge auf. Dabei können die Hohlfaserlagen teilweise überlappend zueinander angeordnet sein und freie Enden der Lagen jeweils in einem nicht überlappenden Bereich von einer benachbarten Lage hervorstehen.

Als eine zentrische Anordnung ist dabei bevorzugt eine Anordnung zentrisch in Bezug auf eine Drehachse der Zentrifuge zu verstehen. Die Anordnung kann z.B. auf einem Drehteller oder einer sonstigen Schleudervorrichtung erfolgen.

Ferner kann das Verpotten in einem einzigen Arbeitsschritt bzw. gegenüber vorbekannten Verfahren in deutlich weniger Arbeitsschritten erfolgen. Dies spart Zeit und/oder Kosten ein, und das Herstellungsverfahren kann auf einfachere Weise automatisiert werden. Es muss nicht wie z.B. bei einem (insbesondere gelegten) Oxygenatormodul mit rechteckigem Querschnitt ein Verpotten jeder einzelnen der vier Seitenflächen erfolgen, oder bei einem gewickelten Oxygenatormodul ein Verpotten von zwei Seiten. Vielmehr kann ein Verpotten von einem zylindrischen, inneren Faserbündel durch Vorsehen eines zylindrischen ringförmigen Verpottungsbereichs radial außen an dem Faserbündel erfolgen. Dabei kann auch bei einer eckigen Außenform mittels der Verpottung eine zylindrische Kavität bereitgestellt werden.

Als ein Verpotten ist dabei bevorzugt ein Vergießen von Fasermatten oder einem alternativen für den Gastransport innerhalb von Blut vorgesehenen Material zu verstehen.

Als ein Modul für eine Mehrfluidfaservorrichtung ist dabei bevorzugt jede ortsfeste Anordnung von Hohlfasern in Verpottungsmasse zu verstehen, welche in einer Vorrichtung mit einer medizintechnischen Funktion eingesetzt werden kann. Hierzu können neben einem Oxygenatormodul z.B. auch Vorrichtungen vom Typ Membrankontaktor oder Membranfilter gezählt werden.

Das Zuführen von Verpottungsmasse erfolgt bevorzugt unter Rotation des Moduls bzw. Oxygenatormoduls. Indem die Verpottungsmasse erst dann zugeführt wird, wenn die Form bereits gedreht wird und eine Zentrifugalkraft vorliegt, kann auf einfache Weise vermieden werden, dass Verpottungsmasse in Kontakt mit Faserbereichen kommt, welche nicht verpottet werden sollen.

Als eine Mehrfluidfaservorrichtung ist dabei bevorzugt eine medizintechnische Vorrichtung zu verstehen, in welcher in einer von Blut durchströmbaren Kavität angeordnete Hohlfasern umströmt werden können, um einen Austausch von Gas oder Energie zwischen dem Blut und einem in den Hohlfasern geführten Gases zu ermöglichen, insbesondere ein Oxygenator, ein Wärmetauscher, ein Hämofilter, ein Dialysegerät, ein arteriellen Filter, oder auch eine kombinierte Vorrichtung mit z.B. sowohl einem Oxygenator als auch einem Wärmetauscher. Als Fluide können auch zwei Gase oder zwei Flüssigkeiten zum Einsatz kommen, die auch von demselben Typ sein können.

In dem Schritt f) muss die Verpottungsmasse nicht notwendigerweise aus allen Formteilen entnommen werden. Vielmehr kann auch ein Deckel einen Teil der Gießform bilden, welcher dann nach dem Verpotten fest mit den Hohlfaserlagen verbunden ist und zusammen mit der Verpottungsmasse aus weiteren Formteilen entnommen wird.

Gemäß einem Aspekt wird die Form als Negativform zum Ausbilden einer Außenmantelfläche der Verpottungsmasse eingesetzt. Hierdurch kann der Verpottung eine bestimmte Außengeometrie verliehen werden. Bevorzugt wird eine zylindrische Außenmantelfläche ausgebildet, indem eine zylindrische Negativform verwendet wird, die konzentrisch um die Drehachse angeordnet wird. Die Außenmantelfläche kann dabei durch die Negativform selbst gebildet werden oder wahlweise auch mittelbar, indem zwischen der Negativform und der Verpottungsmasse eine Sperrflüssigkeit angeordnet wird.

Gemäß einem bevorzugten Aspekt wird im Schritt a) auch mindestens ein Deckel derart in der Form angeordnet, dass in den Schritten d) und e) die Verpottungsmasse den mindestens einen Deckel zum Begrenzen der Kavität fixiert. Das Verpotten der Lagen zusammen mit einem oder mehreren Deckeln liefert ein effizientes Verfahren und kann auch einen einfachen Aufbau des Oxygenators sicherstellen. Ein zusätzlicher (Stütz-)Rahmen ist dabei nicht erforderlich. Die Verpottungsmasse kann dabei zusammen mit dem mindestens einen Deckel eine hermetisch abdichtbare Kavität bilden. Dies ist bei weiteren Arbeitsschritten vorteilhaft, insbesondere bei spanbildenden Arbeitsschritten.

Das rundverpottete Oxygenatormodul kann optional in einem weiteren Arbeitsschritt durch Schneiden bearbeitet werden, insbesondere um der Außenmantelfläche des Oxygenators eine vieleckige oder runde Geometrie zu verleihen. Dabei können auch geschlossene Fasern eingesetzt werden, die durch das Schneiden der Verpottung geöffnet werden können, so dass sie zugänglich sind und mit einem Gas beschickt werden können. Die Hohlfasern können dabei von einem Hersteller auf Spulen geliefert werden. Eine einzelne Hohlfaserlage kann dabei durch einzelne mäanderförmig zu Matten verwirkte Hohlfasern gebildet sein, wobei die Hohlfasern an den Mattenenden bzw. im Randbereich der jeweiligen Lage verschlossen bzw. geknickt sind. Die Vereinzelung zu mehreren Fasern kann durch ein Schneiden nach dem Verpotten erfolgen. In Verbindung mit einer zylindrischen Außenmantelfläche der Verpottung kann ein Verfahren zum Schneiden bzw. Freilegen der Hohlfasern vereinfacht werden, insbesondere weil die Außengeometrie vor dem Schneiden zumindest annähernd bereits in der späteren Sollgeometrie vorliegt. Das Schneiden ermöglicht dabei auch vergleichsweise gerade Schnittflächen.

Bevorzugt wird dem rundverpotteten Oxygenator eine runde Geometrie verliehen, insbesondere indem er (wie ein Drehteil bei einem Drehvorgang auf einer Drehbank) um seine eigene Mittenlängsachse gedreht wird. Die Mittenlängsachse kann dabei der Drehachse der Zentrifuge entsprechen. Dabei kann während der Drehung des Oxygenators eine Schneideinrichtung wie z.B. ein Messer an den Oxygenator herangeführt werden oder der Oxygenator selbst an eine feststehende Schneideinrichtung, um einen Teil der Verpottung außen von dem Oxygenator abzuschälen. Die Schneideinrichtung kann auch selbst um den Oxygenator herum gedreht werden. Die genannten Bewegungen sind auch miteinander kombinierbar. Das Schneiden kann dabei auch ein Schälen der Hohlfasern umfassen, also nicht allein eine Formgebung der Verpottung, sondern auch ein Öffnen der Hohlfasern. Hierbei können ein Vorschub und die Drehgeschwindigkeit eingestellt werden, insbesondere mittels einer Steuereinrichtung, so dass das Abschälen mit einer definierbaren Schälstärke erfolgen kann.

Gemäß einem Aspekt, welcher mit einem der zuvor beschriebenen Aspekte kombiniert werden kann, wird vor dem Schritt c) eine Sperrflüssigkeit an einer Innenmantelfläche der Form angeordnet, welche eine größere Dichte aufweist als die Verpottungsmasse. Hierdurch kann ein Verfahrensschritt zum Freilegen der Fasern vereinfacht oder eingespart werden. Wahlweise werden dabei geschlossene oder offene Hohlfasern verwendet, welche im Bereich von freien Enden der Hohlfasern aus der Verpottungsmasse hervorstehen. Geschlossene Hohlfasern können einfacher freigelegt werden, da eine Verpottungsmasse dabei nicht abgeschält werden muss, und bei offenen Hohlfasern kann ein Schneiden bzw. Freilegen vollständig entfallen.

Aufgrund der größeren Dichte der Sperrflüssigkeit kann die Sperrflüssigkeit beim Drehen der Form durch die Zentrifugalkraft stärker nach außen getrieben als die Verpottungsmasse, so dass die Sperrflüssigkeit und die Verpottungsmasse nicht vermischt werden, sondern bildlich gesprochen wie bei einem Wasser-Öl-Gemisch, bei welchem sich Öl immer an der Oberfläche vom Wasser anordnet. Durch den Dichteunterschied kann z.B. verhindert werden, dass sich eine Emulsion bildet. Als Sperrflüssigkeit können z.B. kristalloide Lösungen mit höherer spezifischer Dichte als Polyurethan bzw. der verwendeten Verpottungsmasse zum Einsatz kommen.

Die Sperrflüssigkeit kann insbesondere während des Drehens der Form zugeführt werden, so dass sie aufgrund von Zentrifugalkräften an der Innenmantelfläche der Form angeordnet wird. Die Menge an Sperrflüssigkeit wird dabei bevorzugt derart eingestellt, dass die freien Enden der Hohlfasern in der Sperrflüssigkeit angeordnet sind. Hierdurch kann sichergestellt werden, dass die freien Enden nicht von Verpottungsmasse verschlossen werden. Die Sperrflüssigkeit wird z.B. unter Rotation des Moduls zugeführt, bevor die Verpottungsmasse zugeführt wird. Hierbei erfolgt eine Zuführung der Verpottungsmasse bevorzugt in axialer Richtung, d.h. nicht radial von der Seite. Wahlweise kann die Sperrflüssigkeit auch nach dem Einbringen von Verpottungsmasse zugeführt werden und die Verpottungsmasse an der Innenmantelfläche der Form verdrängen. Gemäß einer Variante kann die Sperrflüssigkeit auch bereits in der Form vorgesehen sein, bevor die Hohlfaserlagen in der Form angeordnet werden. Hierbei kann bei der wiederholten Herstellung eines Moduls mit der Form eine Sperrflüssigkeit auch in der Form verbleiben und mehrfach verwendet werden.

Gemäß einem Aspekt, welcher mit einem der zuvor beschriebenen Aspekte kombiniert werden kann, wird zwischen dem Schritt e) und dem Schritt f) die Sperrflüssigkeit abgelassen. Hierdurch kann das Modul schneller in der Form trocknen oder aktiv z.B. mittels eines konvektiven Luftstroms getrocknet werden, bevor es entnommen wird. Gemäß einer Variante kann die Sperrflüssigkeit während des Schrittes e) abgelassen werden, insbesondere um ein schnelleres Trocknen bzw. Aushärten zu ermöglichen. Ein Trocknen der Fasern selbst ist dabei nicht erforderlich. Das Aushärten erfolgt für den Fall von Polyurethan als Verpottungsmasse bevorzugt auf chemische Weise unter Bildung von Wärme (exotherme Reaktion), insbesondere bei fortwährendem Drehen. Ein Ablassen während des Schrittes e) erfolgt bevorzugt erst dann, wenn die Verpottungsmasse bereits zumindest bis zu einem gewissen Grad ausgehärtet ist. Ein Ablassen während des Schrittes e) kann auch zu einem schnelleren Aushärten der Verpottungsmasse führen, insbesondere weil auch an einer Außenmantelfläche der Verpottung z.B. eine Luftzirkulation erfolgen kann oder Wärme eingebracht werden kann. Bevorzugt wird die Sperrflüssigkeit während des Schrittes e) aus der Form entfernt, sobald die Verpottungsmasse derart teilweise ausgehärtet ist, dass sie eigenstabil ist. Bevorzugt werden geschlossene Hohlfasern eingesetzt, damit auf einfache Weise sichergestellt werden kann, dass keine Sperrflüssigkeit in die Hohlfasern gelangen kann. Die Sperrflüssigkeit ist bevorzugt biokompatibel. Hierdurch ist es z.B. nicht erforderlich, mit einer anderen Flüssigkeit nachzuspülen.

Gemäß einem Aspekt, welche mit einem der zuvor beschriebenen Aspekte kombiniert werden kann, werden mindestens zwei, bevorzugt mindestens drei, der Hohlfaserlagen in einem Winkel ungleich Null und kleiner als 90 Grad verdreht zueinander, insbesondere verdreht um die Mittenlängsachse, angeordnet, bevorzugt in einem Winkel von zumindest annähernd 45 oder 60 Grad verdreht zueinander. Hierdurch kann das Fasermaterial besonders gut genutzt werden, insbesondere bei einer zylindrischen Kavität.

Gemäß einer Variante wird die Form gedreht, bis die Verpottungsmasse so weit erstarrt ist, dass sie zumindest eigenstabil ist, und dann wird das Oxygenatormodul aus der Form entnommen, ohne dass vorher die Sperrflüssigkeit abgelassen wird. Hierdurch kann mit derselben Form eine große Anzahl von Modulen pro Zeiteinheit hergestellt werden, was die Stückkosten senken kann. Gleichzeitig kann ein Trocknen bzw. Aushärten der Verpottungsmasse vom Verfahrensschritt der Formgebung innerhalb der Form unabhängig durchgeführt werden. Hierdurch können beim Trocknen und Aushärten auf flexiblere Weise Verfahrensparameter wie z.B. eine Temperatur oder eine Luftzirkulation zum Austrocknen eingestellt werden.

Gemäß einer Variante wird das Oxygenatormodul zunächst mit einem oder zwei Deckeln verbunden, bevor es aus der Form entnommen wird. Die Deckel können einen Bestandteil der Verguss-Form bilden.

Die vorliegende Erfindung betrifft ferner die Verwendung eines erfindungsgemäßen Oxygenators in einem extrakorporalen Lungenunterstützungssystem. Der Oxygenator kann zum Therapieren von Menschen oder Tieren oder auch von irgendeinem isolierten (Spender-) Organ verwendet werden. Vitalfunktionen des (Spender-)Organs können z.B. vollständig isoliert von dem Spender oder dem Empfänger (Mensch/Tier) mit dem extrakorporalen Lungenunterstützungssystem aufrechterhalten werden.

Auch offenbart wird die Verwendung einer zylindrischen fluiddichten Negativform zur Formgebung der Außenmantelfläche einer Verpottung eines Moduls für eine Mehrfluidfaservorrichtung, vorzugsweise eines erfindungsgemäßen Oxygenatormoduls.

In den nachfolgenden Figuren wird die Erfindung anhand von Ausführungsbeispielen näher erläutert, wobei unterschiedliche Komponenten für einen erfindungsgemäßen Oxygenator gezeigt sind, zum einen unterschiedliche Modul, zum anderen unterschiedliche Beströmungseinrichtungen (Drallverteiler und Aspektverteiler). Werden einzelne Bezugszeichen im Zusammenhang mit einzelnen Figuren nicht explizit erläutert, wird hiermit jeweils auf die weiteren Figuren verwiesen. Es zeigen:
- Fig. 1: in einer schematischen Draufsicht ein Oxygenatormodul gemäß dem Stand der Technik, welches in einer Form auf einer Zentrifuge exzentrisch zu einer Drehachse der Zentrifuge angeordnet ist;
- Fig. 2a: in einer schematischen Draufsicht eine Hohlfasermatte mit langen Fasern, welche zum Bilden eines Faserbündels für ein Oxygenatormodul eines erfindungsgemäßen Oxygenators geeignet ist;
- Fig. 2b: in einer schematischen Draufsicht eine Hohlfasermatte mit langen Fasern, die mit einer weiteren Hohlfasermatte mit langen Fasern zum Bilden eines Faserbündels für ein Oxygenatormodul eines erfindungsgemäßen Oxygenators gefaltet werden kann;
- Fig. 2c: in einer schematischen Draufsicht ein aus den beiden in Fig. 2b gezeigten Hohlfasermatten gebildetes Faserbündel;
- Fig. 3a: in einer schematischen Draufsicht eine Hohlfasermatte mit kurzen Fasern, die zu Faserpaketen zusammengefasst sind, welche beabstandet zueinander angeordnet sind, wobei die Hohlfasermatte zum Bilden eines Faserbündels für ein Oxygenatormodul eines erfindungsgemäßen Oxygenators geeignet ist;
- Fig. 3b: in einer schematischen Draufsicht eine Hohlfasermatte mit kurzen Fasern, die mit einer weiteren Hohlfasermatte mit kurzen Fasern zum Bilden eines Faserbündels für ein Oxygenatormodul eines erfindungsgemäßen Oxygenators gefaltet werden kann;
- Fig. 3c: in einer schematischen Draufsicht ein aus den beiden in Fig. 3b gezeigten Hohlfasermatten gebildetes Faserbündel;
- Fig. 4a: in einer schematischen Draufsicht ein in einer Form zum Herstellen eines Oxygenatormoduls für einen Oxygenator gemäß einem Ausführungsbeispiel der Erfindung angeordnetes Faserbündel mit Hohlfaserlagen mit rechteckiger Grundform mit unterschiedlicher Seitenlänge;
- Fig. 4b: in einer schematischen Draufsicht die in Fig. 4a gezeigte Form mit darin eingebrachter und an einer Innenmantelfläche der Form angeordneter Verpottungsmasse;
- Fig. 5a: in einer schematischen Draufsicht das mittels der in der Fig. 4b gezeigten Form hergestellte Oxygenatormodul;
- Fig. 5b: in einer schematischen Draufsicht das in der Fig. 5a gezeigte Oxygenatormodul in einem Verfahrensschritt, in welchem mittels einer Schneideinrichtung die Verpottung des Oxygenatormoduls bearbeitet wird;
- Fig. 5c: in einer schematischen Draufsicht das durch Schneiden bearbeitete Oxygenatormodul der Fig. 5b;
- Fig. 6a: in einer schematischen Draufsicht ein in einer Form zum Herstellen eines Oxygenatormoduls für einen Oxygenator gemäß einem Ausführungsbeispiel der Erfindung angeordnetes Faserbündel mit Hohlfaserlagen mit rechteckiger Grundform mit unterschiedlicher Seitenlänge;
- Fig. 6b: in einer schematischen Draufsicht die in Fig. 6a gezeigte Form mit darin eingebrachter Verpottungsmasse sowie darin eingebrachter Sperrflüssigkeit, die an einer Innenmantelfläche der Form außerhalb von der Verpottungsmasse angeordnet ist;
- Fig. 6c: in einer schematischen Draufsicht das Oxygenatormodul der Fig. 6b mit einer mittels der Sperrflüssigkeit in Form gebrachten Verpottung, aus welcher die Hohlfaserlagen hervorstehen;
- Fig. 7a: in einer schematischen perspektivischen Ansicht einen Oxygenator mit einem Oxygenatormodul gemäß einem Ausführungsbeispiel der Erfindung;
- Fig. 7b: in einer schematischen Schnittansicht den in der Fig. 7a gezeigten Oxygenator;
- Fig. 8: in einer schematischen perspektivischen Ansicht einen Drallverteiler für einen Oxygenator gemäß einem Ausführungsbeispiel der Erfindung;
- Fig. 9a: in einer schematischen perspektivischen Ansicht einen Aspektverteiler für einen Oxygenator gemäß einem Ausführungsbeispiel der Erfindung;
- Fig. 9b: in einer weiteren schematischen perspektivischen Ansicht den in der Fig. 9a gezeigten Aspektverteiler;
- Fig. 10: in einer schematischen perspektivischen Ansicht einen Tangentialverteiler für einen Oxygenator gemäß einem Ausführungsbeispiel der Erfindung;
- Fig. 11: in einer schematischen perspektivischen Ansicht einen sechseckigen Oxygenator mit einem (hexagonalen) Deckel gemäß einem weiteren Ausführungsbeispiel der Erfindung;
- Fig. 12A, 12B: jeweils in einer schematischen Draufsicht Komponenten eines Oxygenatormoduls für einen Oxygenator gemäß einem weiteren Ausführungsbeispiel der Erfindung in einer Anordnung auf einem Deckel;
- Fig. 13A, 13B: in einer schematischen Draufsicht und in einer perspektivischen Ansicht einen Oxygenator gemäß einem Ausführungsbeispiel der Erfindung mit dem in Fig. 11 gezeigten sechseckigen (hexagonalen) Deckel in einer Anordnung in einem Gehäuse des Oxygenators; und
- Fig. 14: in einer schematischen perspektivischen Explosions-Ansicht einzelnen Komponenten eines Oxygenators gemäß einem Ausführungsbeispiel der Erfindung, insbesondere auch mit dem in Fig. 11 gezeigten Deckel sowie den in Fig. 12A, 12B gezeigten Komponenten des Oxygenatormoduls.

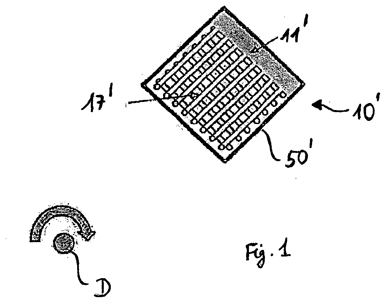

In der Fig. 1 ist ein in einer Form 50' angeordnetes Faserbündel 17' gezeigt, wobei die Form 50' auf einer Zentrifuge (nicht dargestellt) angeordnet ist und um eine Drehachse D der Zentrifuge rotiert wird. Hierdurch soll schrittweise ein Oxygenatormodul 10' hergestellt werden. Die Fig. 1 zeigt den Stand der Technik. Es wird eine rechteckige, insbesondere quadratische Form 17' verwendet. Es wird zunächst eine der vier Seitenflächen des Oxygenatormoduls 10' mit einer Verpottung 11' versehen, indem Verpottungsmasse für eine der vier Seiten des Faserbündels 17' in die Form 50' eingebracht wird und die Form 50' um die Drehachse D rotiert wird. Eine aufgrund der Drehung wirkende Zentrifugalkraft greift an der Verpottungsmasse an und treibt diese an den radial am weitesten außenliegenden Punkt. Dies ist zumindest in grober Annäherung die (komplette) radial außenliegende Seitenfläche der Form 50'. Die Außenfläche der Verpottung 11' nimmt dabei die Geometrie einer Innenfläche der radial außenliegenden Seitenfläche der Form 50' an. Dabei ist die Form 50' exzentrisch zu der Drehachse D angeordnet, um eine im Wesentlichen ebene Innenwandungsfläche zu erhalten. Sobald die Verpottungsmasse eigenstabil ist, also zumindest etwas erstarrt ist, kann die Drehung unterbrochen werden und die Form 50' um 90 Grad um die eigene Mittenlängsachse verdreht angeordnet werden, um eine weitere der Seitenflächen zu verpotten. Dabei ist das Faserbündel 17' zwischen zwei Blutdeckeln (nicht dargestellt) in axialer Richtung verspannt, so dass vermieden werden kann, dass sich das Faserbündel 17' relativ zu der Längsachse verdreht. Die Schritte können wiederholt werden, bis alle vier Seiten verpottet sind und das Faserbündel vollständig in der Verpottung 11' fixiert ist.

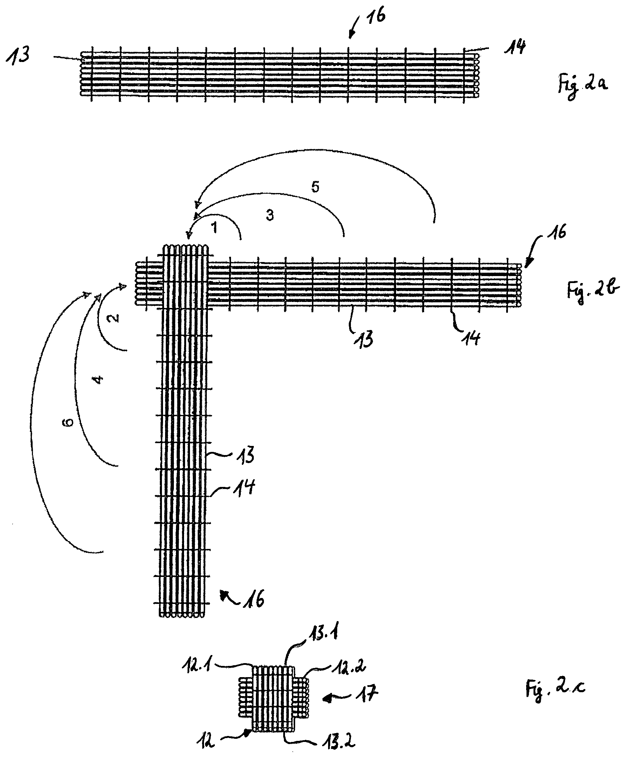

In der Fig. 2a ist eine Hohlfasermatte 16 gezeigt, welche aus einer Vielzahl, insbesondere acht, nebeneinander angeordneter Fasern 13 gebildet ist, wobei die Fasern 13 über Kettfäden 14 miteinander verbunden sind. Die Kettfäden 14 erstrecken sich orthogonal zu den Fasern 13. Die Anzahl der Fasern kann bis zu 200 betragen, wobei in der Fig. 2a aus Gründen der Übersichtlichkeit nur acht Fasern dargestellt sind.

In der Fig. 2b ist eine weitere Hohlfasermatte 16 gezeigt, die um 90 Grad verdreht zu der anderen Hohlfasermatte 16 auf dieser angeordnet ist und denselben Aufbau aufweist. Die beiden Hohlfasermatten 16 können zu einem Faserbündel gefaltet werden, indem zunächst die untere Hohlfasermatte umgefaltet wird (Pfeil 1) und dann weitere Faltungen erfolgen (Pfeile 2, 3, 4, 5 und 6).

In der Fig. 2c ist ein aus den in der Fig. 2b gezeigten Hohlfasermatten 16 gefaltetes Faserbündel 17 gezeigt, wobei zueinander um zumindest annähernd 90 Grad verdrehte Hohlfaserlagen 12.1, 12.2 gebildet wurden, welche mit freien Enden 13.1, 13.2 der jeweiligen Fasern 13 voneinander hervorstehen. Die Hohlfaserlagen 12.1, 12.2 überlappen sich alle in einem (insbesondere quadratischen) Kernbereich. Ferner gibt es Bereiche, in welchen sich nur diejenigen Hohlfaserlagen 12.1 bzw. 12.2 überlappen, bei welchen die Fasern 13 in derselben Richtung ausgerichtet sind. In diesen Bereichen können die Fasern 13 der einen Lage 12.1 an ihren Enden 13.1, 13.2 bearbeitet werden, insbesondere geöffnet werden, ohne dass die Fasern einer anderen Lage 12.2 beschädigt werden.

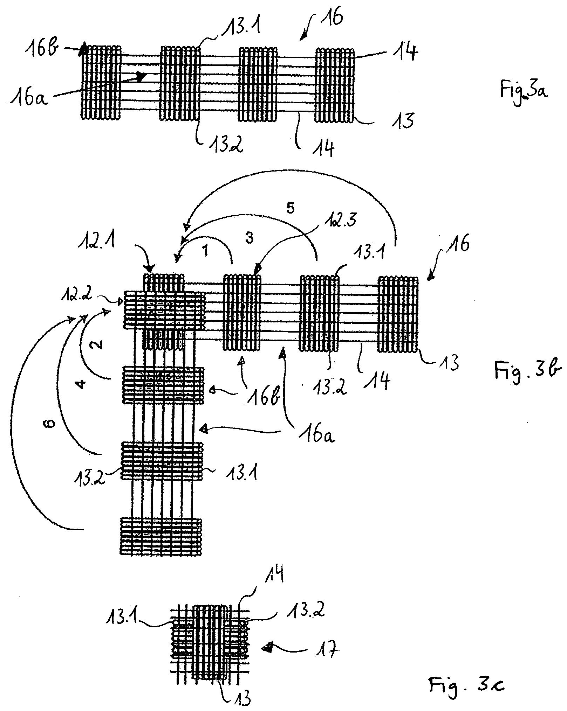

In der Fig. 3a ist eine Hohlfasermatte 16 gezeigt, welche aus einer Vielzahl von Hohlfaserpaketen 16b gebildet ist, die in einem Abstand zueinander vorgesehen sind, so dass sich Leerstellen 16b ergeben. Jedes Hohlfaserpaket 16b weist eine Vielzahl von Fasern 13 auf, insbesondere bis zu 200 nebeneinander angeordneter Fasern 13 (exemplarisch dargestellt sind acht Fasern), wobei die Fasern 13 bzw. Hohlfaserpakete 16b über Kettfäden 14 miteinander verbunden sind. Die Kettfäden 14 erstrecken sich senkrecht zu den Fasern 13 und sind länger als die Fasern 13.

In der Fig. 3b ist eine weitere Hohlfasermatte 16 gezeigt, die um 90 Grad verdreht zu der anderen Hohlfasermatte 16 auf dieser angeordnet ist und denselben Aufbau aufweist. Die beiden Hohlfasermatten 16 können zu einem Faserbündel gefaltet werden, indem zunächst die untere Hohlfasermatte umgefaltet wird (Pfeil 1) und dann weitere Faltungen erfolgen (Pfeile 2, 3, 4, 5 und 6). Dabei werden mehrere Hohlfaserlagen 12.1, 12.2, 12.3 gebildet, welche jeweils Fasern 13 aufweisen, die unabhängig voneinander angeordnet sind, also nicht miteinander verbunden sind. Die freien Enden 13.1, 13.2 der Fasern 13 können dabei offen oder geschlossen sein.

In der Fig. 3c ist ein aus den in der Fig. 3b gezeigten Hohlfasermatten 16 gefaltetes Faserbündel 17 gezeigt. Die Kettfäden 14 liegen über einen Abschnitt mit quadratischer Grundform vor, also auch in Bereichen, in welchen keine Fasern 13 vorliegen. Die Kettfäden 14 können dabei aus dem Faserbündel 17 herausgetrennt werden, nachdem es gefaltet wurde.

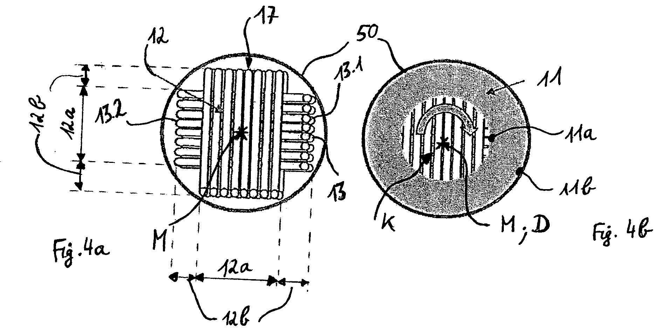

In der Fig. 4a ist ein Hohlfaserbündel 17 gezeigt, welches in einer Form 50 zum Herstellen einer Verpottung angeordnet ist. In dieser Form 50 kann das Hohlfaserbündel 17 um eine Mittenlängsachse M des Hohlfaserbündels gedreht werden, wofür es auf einer Zentrifuge (nicht dargestellt) angeordnet werden kann. Dabei kann das Hohlfaserbündel 17 in der Form 50 mittels zwei Blutdeckeln bzw. Deckeln (nicht dargestellt) relativ zu der Form 50 fixiert werden, solange noch keine Verpottungsmasse in die Form eingebracht ist. Die einzelnen Hohlfaserlagen 12 sind um 90 Grad verdreht zueinander angeordnet und weisen jeweils eine rechteckige Grundform mit unterschiedlicher Seitenlänge auf, so dass überlappende Kernbereiche 12a und hervorstehende, nicht überlappende Abschnitte 12b gebildet sind. Hierdurch können die freien Enden 13.1, 13.2 der Fasern 13 jeweils einer Hohlfaserlage 12 von Verpottungsmasse eingefasst werden, insbesondere oberhalb und unterhalb der Hohlfaserlage 12 (in Bezug auf die Mittenlängsachse M). Dies liefert eine gute Verankerung bzw. Fixierung der Fasern in der Verpottungsmasse und auch eine gute Genauigkeit in Bezug auf die Ausrichtung der Faserenden. Zwischen den einzelnen Lagen 12 kann in den nicht überlappenden Abschnitten 12b jeweils Verpottungsmasse vorgesehen werden.

Gemäß einer Variante können die in der Fig. 4a gezeigten Hohlfaserlagen 12 auch in einem Winkel von 45 oder 60 Grad zueinander angeordnet sein, insbesondere aufeinanderliegende Lagen jeweils um 45 oder 60 Grad verdreht zur benachbarten Lage. Bei dieser Anordnung der Lagen kann die Oberfläche der Fasern im Vergleich zur 90 Grad-Anordnung (orthogonale Anordnung) noch besser ausgenutzt werden.

In der Fig. 4b ist das Hohlfaserbündel 17 in einem um eine Drehachse D einer Zentrifuge (hier entsprechend der Mittenlängsachse M) rotierenden Zustand gezeigt, wobei Verpottungsmasse 11 in die Form 50 eingebracht wurde. Das Hohlfaserbündel 17 und/oder die Form 50 sind dabei auf der Zentrifuge, insbesondere einem Drehteller, fixiert. Das Hohlfaserbündel 17 ist dabei in der Form 50 fixiert. Durch eine aufgrund der Drehung auf die Verpottungsmasse 11 wirkende Zentrifugalkraft wird die Verpottungsmasse 11 an eine Innenmantelfläche der Form 50 getrieben, so dass der Verpottungsmasse 11 eine Außenmantelfläche 11b verliehen wird, welche ein Negativ der Innenmantelfläche der Form 50 ist. Gleichzeitig bildet sich eine im Querschnitt wie dargestellt im Wesentlichen kreisförmige Innenmantelfläche 11a aus, die in Bezug auf die Mittenlängsachse M zumindest im Wesentlichen zylindrisch ist. Im beschriebenen Fall entsteht eine ringförmige, in Bezug auf die Mittenlängsachse M zumindest im Wesentlichen rohrartige Verpottung, welche eine zumindest im Wesentlichen zylindrische Kavität K umgrenzt. Durch diese Kavität K kann z.B. Blut strömen.

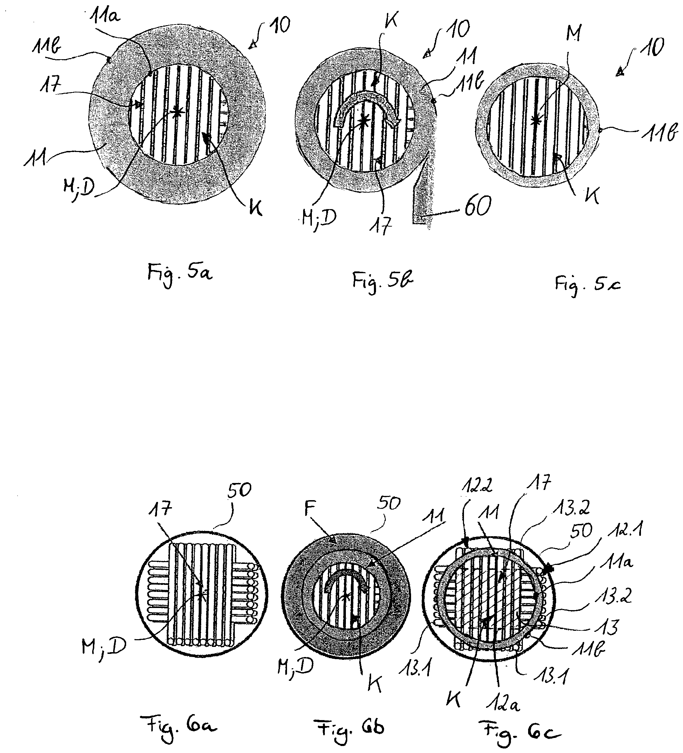

Die Fig. 5a zeigt das Faserbündel 17 in einem aus der Form (nicht dargestellt) entnommenen Zustand, in welchem die Verpottungsmasse 11 erstarrt ist und das Faserbündel 17 bereits in der Verpottungsmasse 11 fixiert ist. Für den Fall, dass als Verpottungsmasse Polyurethan verwendet wird, ist bereits ein Aushärten der Verpottungsmasse erfolgt. Es wird ein rundverpottetes Oxygenatormodul 10 bereitgestellt. Die Form kann von dem Faserbündel 17 entfernt worden sein, so dass das Faserbündel 17 weiterhin auf einem Drehteller einer Zentrifuge angeordnet bzw. fixiert sein kann.

In der Fig. 5b ist gezeigt, dass das rundverpottete Oxygenatormodul 10 mittels einer Schneideinrichtung 60 weiterbearbeitet werden kann, z.B. um die Außenmantelfläche 11b der Verpottung 11 in eine bestimmte Geometrie zu bringen oder mit einer bestimmten Struktur, Rauhigkeit oder Güte zu versehen. Dabei kann das Oxygenatormodul 10 auf einer Zentrifuge angeordnet bleiben und die Schneideinrichtung 60 kann wie eine Art Drehmeißel an das rotierende Oxygenatormodul 10 geführt werden. Das (Ab-)Schneiden von Faserenden und/oder ein Abdrehen des Verpottungsmaterials 11 (insbesondere durch denselben Schneidvorgang) kann dabei z.B. auch dazu erfolgen, die Faserenden des Faserbündels 17 freizulegen.

In der Fig. 5c ist das Oxygenatormodul 10 mit einer nachbearbeiteten Rundverpottung gezeigt. Die Außenmantelfläche 11b ist im Querschnitt kreisförmige, die Verpottung selbst ringförmig bzw. entlang der Mittenlängsachse rohrartig ausgebildet.

In der Fig. 6a ist ein Hohlfaserbündel 17 gezeigt, welches wie das in der Fig. 4a gezeigte Hohlfaserbündel in einer Form 50 zum Herstellen einer Verpottung angeordnet ist. Gemäß einer Variante können die in der Fig. 6a gezeigten Hohlfaserlagen des Hohlfaserbündels 17 auch in einem Winkel von 45 oder 60 Grad zueinander angeordnet sein, insbesondere aufeinanderliegende Lagen jeweils um 45 oder 60 Grad verdreht zur benachbarten Lage.

In der Fig. 6b ist das Hohlfaserbündel 17 in einem um eine Drehachse D einer Zentrifuge (hier entsprechend der Mittenlängsachse M) rotierenden Zustand gezeigt, wobei sowohl Verpottungsmasse 11 als auch eine Sperrflüssigkeit F in die Form 50 eingebracht wurde. Die Sperrflüssigkeit F wird bei Rotation der Form 50 um die Drehachse D außen von der Verpottungsmasse 11 angeordnet, insbesondere aufgrund ihrer höheren Dichte (relativen, volumenspezifischen Masse). Die Sperrflüssigkeit F kommt an einer Innenmantelfläche der Form 50 zur Anlage.

In der Fig. 6c ist die Sperrflüssigkeit nach einem Erstarren der Verpottungsmasse 11 aus der Form 50 entfernt worden, so dass eine Verpottung gebildet ist, welche radial beabstandet von der Innenmantelfläche der Form 50 angeordnet ist. Die freien Enden 13.1, 13.2 der Fasern 13 stehen radial außen aus der Verpottung hervor. Es ist erkennbar, dass im in der Fig. 6b gezeigten Verfahrensschritt ausreichend Sperrflüssigkeit F eingebracht wurde, dass die Verpottung radial innerhalb von allen freien Enden 13.1, 13.2 angeordnet ist. Die Außenmantelfläche 11b der Verpottung weist einen kleineren Durchmesser auf als die Fasern 13 in ihrer Erstreckungsrichtung. Die Verpottung ist als dünnwandiges Rohr innerhalb des Faserbündels 17 in das Faserbündel 17 integriert, d.h., sie ist durch verhältnismäßig wenig Verpottungsmaterial 11 gebildet. Bevorzugt umgrenzt die Verpottung einen Kernbereich, in welchem eine erste Hohlfaserlage 12.1 und eine zweite Hohlfaserlage 12.2 einander vollständig überlappen. Mit anderen Worten weist die Innenmantelfläche 11a der Verpottung 11 bevorzugt einen Innendurchmesser auf, welcher den Abmessungen des quadratischen Kernbereichs 12a (diagonal gestrichen dargestellt) entspricht. Hierdurch kann die Verpottung 11 auch in den vier Eckbereichen vorgesehen sein, in welchen die Lagen 12.1, 12.2 jeweils mit ihren nicht überlappenden, hervorstehenden Abschnitten aneinandergrenzen. Dadurch kann das Faserbündel 17 zum einen mit guter Stabilität in der Verpottung 11 verankert/fixiert werden, zum anderen kann vermieden werden, dass sich ein Strömungspfad mit besonders niedrigem Strömungswiderstand in diesen Eckbereichen bildet (was der Fall wäre, wenn der Innendurchmesser der Verpottung größer wäre als die Länge der Diagonalen des quadratischen Kernbereichs 12a).

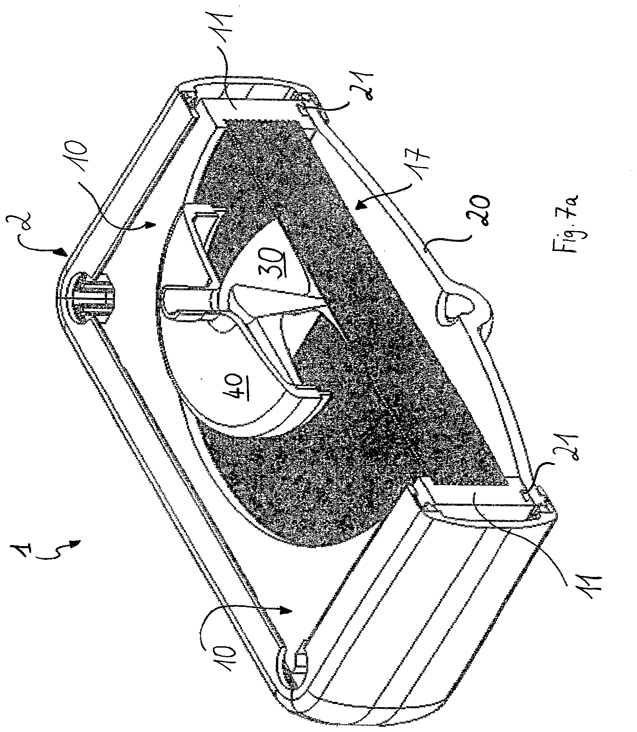

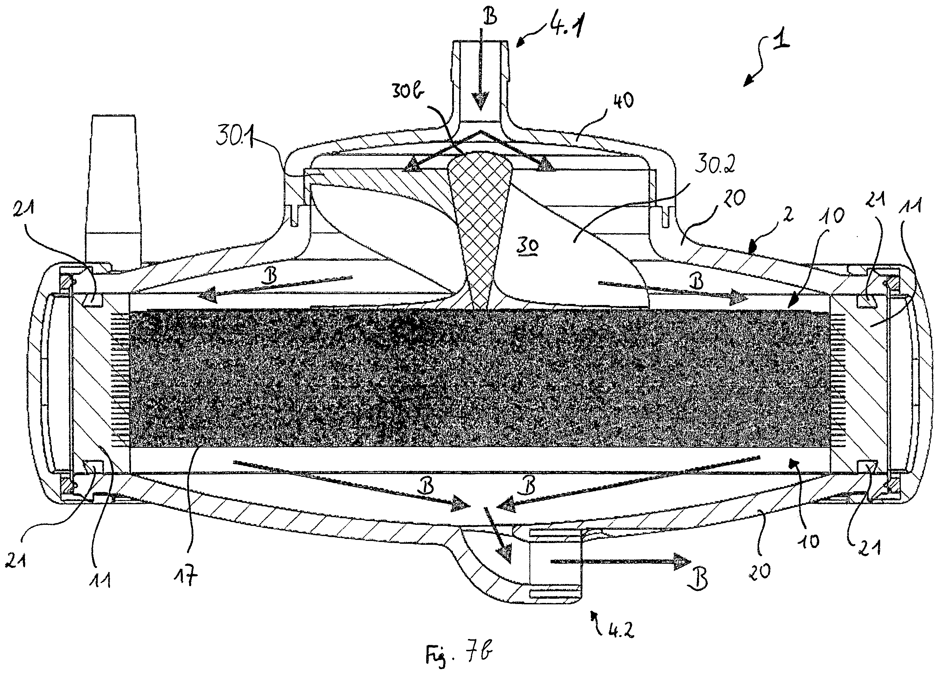

In der Fig. 7a ist ein Oxygenator 1 gezeigt, welcher ein Oxygenatormodul 10 aufweist, das in einem Gehäuse 2 des Oxygenators 1 fixiert ist. Eine Verpottung 11 des Oxygenatormoduls 10 ist mit einem Deckel 20 mittels Befestigungsmitteln 21 verbunden. Die Verpottung 11 weist eine zylindrische Innenmantelfläche auf und definiert damit eine zylindrische Kavität, welche auf homogene Weise von Blut durchströmbar ist. Ein Blutstrom kann mittels eines Aspektverteilers 40 (ohne Drallelemente) und eines Drallverteilers 30 mit flügelartigen Drallelementen auf ein Hohlfaserbündel 17 verteilt werden. Ein weiterer Deckel (nicht dargestellt; vergleiche Deckel 20 in Fig. 7b) ist zwischen dem Oxygenatormodul 10 und dem Aspektverteiler 40 angeordnet. Der Blutstrom durchströmt das Hohlfaserbündel 17 dadurch auf gleichförmige Weise. Das Hohlfaserbündel 17 weist einen Durchmesser auf, welcher größer ist als ein Durchmesser des Aspektverteilers 40 bzw. des Drallverteilers 30.

In der Fig. 7b ist der Blutstrom B gezeigt, in welcher Form er von einem Bluteinlass 4.1 zu einem Blutauslass 4.2 des Gehäuses 2 das Oxygenatormodul 10 durchströmen kann. Bevor der Blutstrom B auf den Drallverteiler 30 trifft, wird er durch den Aspektverteiler 40 aufgeweitet. Der Drallverteiler 30 weist eine Prallfläche 30b auf, welche unterhalb vom Bluteinlass 4.1 angeordnet ist und Teil eines zentrisch angeordneten Dorns bzw. rotationssymmetrischen Umströmungskörpers ist, von welchem sich radial nach außen Flügel 30.1, 30.2 erstrecken. Der Drallverteiler 30 lenkt den Blutstrom B seitlich radial nach außen um, bevor der Blutstrom B auf das Hohlfaserbündel 17 des Oxygenatormoduls 10 trifft. Das Oxygenatormodul 10 ist mittels der Verpottung 11 an Befestigungsmittel 21 des entsprechenden Deckels 20 gekuppelt. Stromauf von dem Oxygenatormodul 10 ist der entsprechende Deckel 20 zwischen dem Aspektverteiler 40 und der Verpottung 11 angeordnet. Ein Bluteinlass 4.1 wird durch den Aspektverteiler 40 bereitgestellt, und ein Blutauslass 4.2 wird durch den unteren Deckel 20 bereitgestellt.

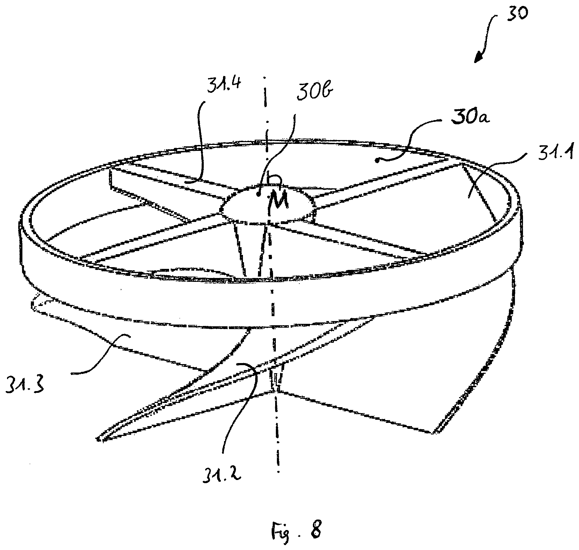

In der Fig. 8 ist ein Drallverteiler 30 gezeigt, welcher zur (insbesondere statischen, starren, also unbewegten) Anordnung in einem Oxygenator gemäß der Fig. 7a, 7b ausgebildet ist, insbesondere stromauf eines Oxygenatormoduls, und welcher eine konzentrisch um eine Mittenlängsachse M angeordnete Prallfläche 30b aufweist, von welcher sich vier Drallelemente bzw. Flügel 31.1, 31.2, 31.3, 31.4 radial nach außen bis zu einer Innenmantelfläche 30a erstrecken, an welcher sie jeweils zumindest annähernd orthogonal angrenzen. Die Innenmantelfläche 30a ist zumindest annähernd konzentrisch um eine Mittenlängsachse M ausgebildet und weist eine im Querschnitt kreisrunde Geometrie auf. Die Flügel 31.1, 31.2, 31.3, 31.4 gehen im Bereich der Mittenlängsachse M ineinander über, so dass ein Blutstrom in unterschiedliche Teilströme aufgetrennt wird. Jedem Teilstrom kann eine neue Strömungsrichtung verliehen werden, die bevorzugt mit einer Umlenkung jeweils im Bereich von 90 Grad einhergeht. Hierdurch kann ein Blutstrom eine Kavität eines Oxygenatormoduls derart durchströmen, dass eine möglichst große Oberfläche von in der Kavität angeordneten Hohlfasern umströmt wird, was einen effektiven Gasaustausch sicherstellen kann. Mittels der Flügel 31.1, 31.2, 31.3, 31.4 kann ein Blutstrom besonders stark umgelenkt werden. Aufgrund der gleichartigen Ausgestaltung der Flügel 31.1, 31.2, 31.3, 31.4 kann den Teilströmen ein zu den anderen Teilströmen jeweils weitgehend vergleichbarer Drall verliehen werden, so dass die Teilströme innerhalb der Kavität die Fasern jeweils auf gleichartige Weise, insbesondere mit gleichem Anströmwinkel und gleicher Strömungsgeschwindigkeit, durchströmen und sich nach einer gewissen Strecke auch wieder zu einem Blutstrom vereinigen können.

Mittels der Prallfläche 30b kann eine Aufteilung eines Blutstroms auf homogene Weise auf die vier durch die Flügel festgelegten Teilbereiche erfolgen. Die Prallfläche 30b weist eine entgegen der Strömungsrichtung konvexe Wölbung auf, was ermöglicht, das Umlenken des Blutstroms auf besonders blutschonende Weise vorzunehmen.

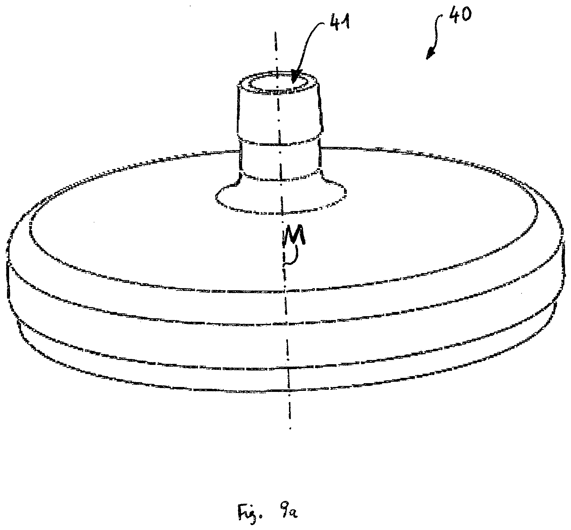

In der Fig. 9a ist ein Aspektverteiler 40 gezeigt, welcher zur (insbesondere statischen, starren, also unbewegten) Anordnung in einem Oxygenator gemäß der Fig. 7-, 8a ausgebildet ist, insbesondere stromauf eines Oxygenatormoduls, und welcher eine konzentrisch um eine Mittenlängsachse M angeordnete Innenmantelfläche 40a aufweist. Der Aspektverteiler 40 weist eine zentrische Blende 41 auf, durch welche ein Blutstrom strömen kann, um dann an der Innenmantelfläche 40a entlang zu strömen und sich gemäß dem Verlauf der Innenmantelfläche 40a in Bezug auf die Mittenlängsachse M aufzuweiten. Der Aspektverteiler 40 ist rotationssymmetrisch um eine Mittenlängsachse M ausgebildet. Der Aspektverteiler 40 kann wahlweise allein oder in Verbindung mit einem Drallverteiler eingesetzt werden.

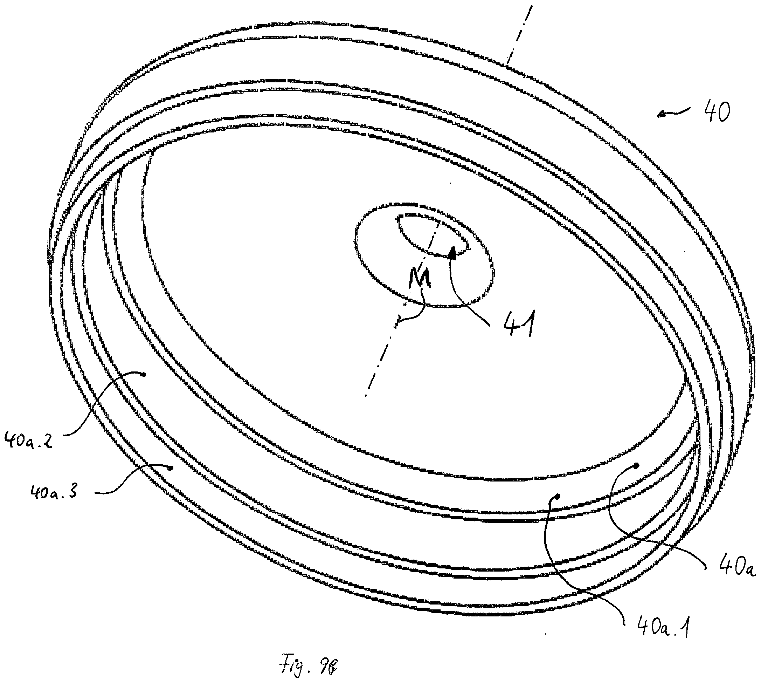

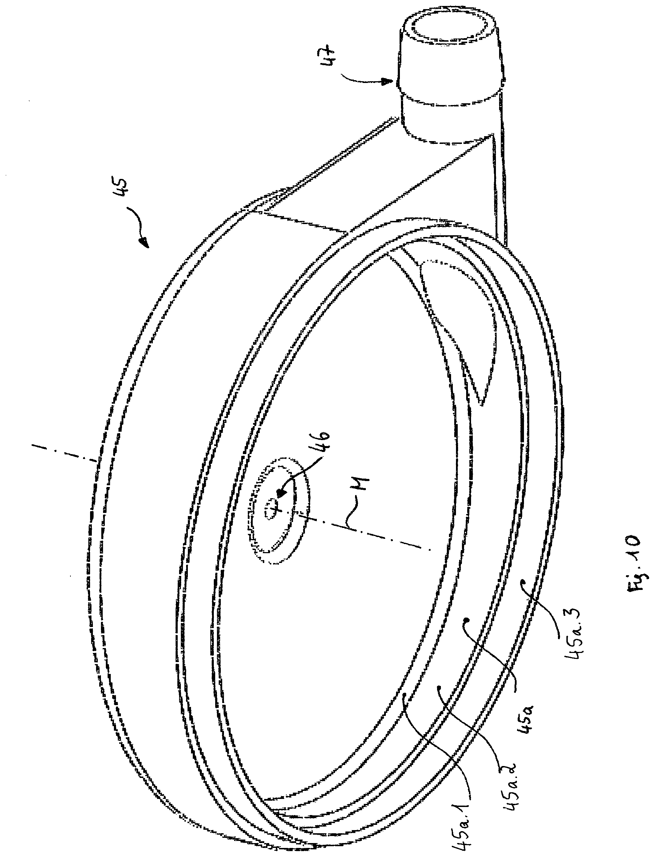

In der Fig. 9b ist gezeigt, dass eine Innenmantelfläche 40a des Aspektverteilers 40 eine gestufte Geometrie aufweisen kann. Die Innenmantelfläche 40a ist in eine in Strömungsrichtung erste Innenmantelfläche 40a.1, eine zweite Innenmantelfläche 40a.2 und eine dritte Innenmantelfläche 40a.3 unterteilt, welche jeweils einen größeren Radius aufweisen als die vorhergehende Innenmantelfläche. Hierdurch kann der Aspektverteiler 40 auf zweckdienliche Weise mit einem Deckel und einem Drallverteiler gekoppelt bzw. verbunden werden, wie in Fig. 8b dargestellt. Der Drallverteiler kann an der zweiten Innenmantelfläche 40a.2 zentrisch zur Anlage kommen, und der Aspektverteiler 40 selbst kann mittels der dritten Innenmantelfläche 40a.3 in Bezug auf einen Deckel zentriert werden. Ein Blutstrom gelangt dabei nur mit der ersten Innenmantelfläche 40a.1 in Kontakt. Der in Fig. 10b gezeigte Aspektverteiler 40 kann wahlweise auch mit einem oder mehreren Drallelementen versehen werden und zu einem Drallverteiler weitergebildet werden.

In der Fig. 10 ist ein Tangentialverteiler 45 gezeigt, welcher eine Blende 46 und einen tangentialen Einlass 47 sowie eine konzentrisch um eine Mittenlängsachse M angeordnete Innenmantelfläche 45a aufweist. Der Tangentialverteiler 45 weist keine Drallelemente auf. Die Innenmantelfläche 45a ist in eine in Strömungsrichtung erste Innenmantelfläche 45a.1, eine zweite Innenmantelfläche 45a.2 und eine dritte Innenmantelfläche 45a.3 unterteilt, wobei die erste Innenmantelfläche 45a.1 und die zweite Innenmantelfläche 45a.2 bevorzugt denselben Radius aufweisen. Der Einlass 47 mündet an der zweiten Innenmantelfläche 45a.2 und damit z.B. in den Bereich eines Drallverteilers, mit welchem der Tangentialverteiler 45 wahlweise kuppelbar ist. Hierdurch kann die Umlenkung des Blutstroms auf effektivere Weise erfolgen. Durch die tangentiale Einströmung und die damit einhergehende Rotation des Blutstroms kann vermieden werden, dass sich Luftblasen bilden, die nicht entweichen können. Für den Fall, dass Luftblasen überhaupt auftreten, können diese sich in der Mitte des Verteilers sammeln und insbesondere in Richtung der Blende 46 entweichen. Ein Zufuhr-Blutstrom kann dabei wahlweise teilweise durch die Blende 46 geleitet werden, welche im Vergleich zu einer Blende eines Aspektverteilers bevorzugt kleiner ausgeführt ist.

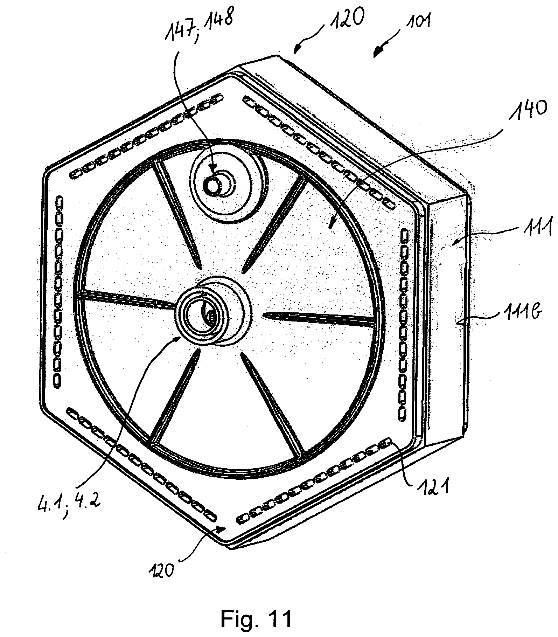

In der Fig. 11 ist ein Oxygenator 101 gezeigt, welcher eine hexagonale Verpottung 111 aufweist. Die Verpottung 111 weist eine Außenmantelfläche 111b mit sechs zumindest annähernd geradflächigen, ebenen Flächenabschnitten auf. Die hexagonale Außengeometrie der Verpottung 111 kann insbesondere durch Schneiden hergestellt werden.

Ferner ist ein Deckel 120 gezeigt, welcher mit der Verpottung 111 verbunden ist. Der Deckel 120 weist eine hexagonale Geometrie mit sechs gleichseitigen Außenmantelflächenabschnitten auf. Am Deckel 120 sind Befestigungsmittel 121 angeordnet, mittels welchen der Deckel 120 mit der Verpottung 111 (der ausgehärteten/aushärtenden Verpottungsmasse) verbunden werden kann. Die Befestigungsmittel 121 können z.B. als Rastnasen, hervorstehende Absätze oder Kanten, und/oder als Ausnehmungen ausgebildet sein. Die Befestigungsmittel 121 können beim Vergießen der Verpottungsmasse in die Verpottung 111 eingebettet werden. Die Befestigungsmittel 121 erstrecken sich jeweils längs entlang eines jeweiligen Außenmantelflächenabschnitts. Unterhalb von der Verpottung 111 ist ein weiterer Deckel 120 vorgesehen. Zwischen den Deckeln 120 ist mindestens ein Oxygenatormodul (nicht dargestellt) angeordnet.

Im Deckel 120 ist ein Verteiler oder Verteilerabschnitt 140 ausgebildet. Der Verteiler 140 wird bevorzugt durch den Deckel 120 gebildet und kann radial ausgerichtete Verstärkungsstege aufweisen. Der Deckel 120 kann wahlweise auch eine geometrisch mit dem Verteiler 140 korrespondierende Öffnung oder Aufnahme aufweisen, in welcher ein separater Verteiler angeordnet werden kann. Am Verteiler 140 ist ein zentrisch angeordneter Bluteinlass 4.1 oder Blutauslass 4.2 vorgesehen. Ferner weist der Verteiler 140 einen seitlich angeordneten Einlass 147 oder Auslass 148 auf, insbesondere eine Entlüftung, welche am höchsten Punkt des Oxygenators 101 angeordnet ist.

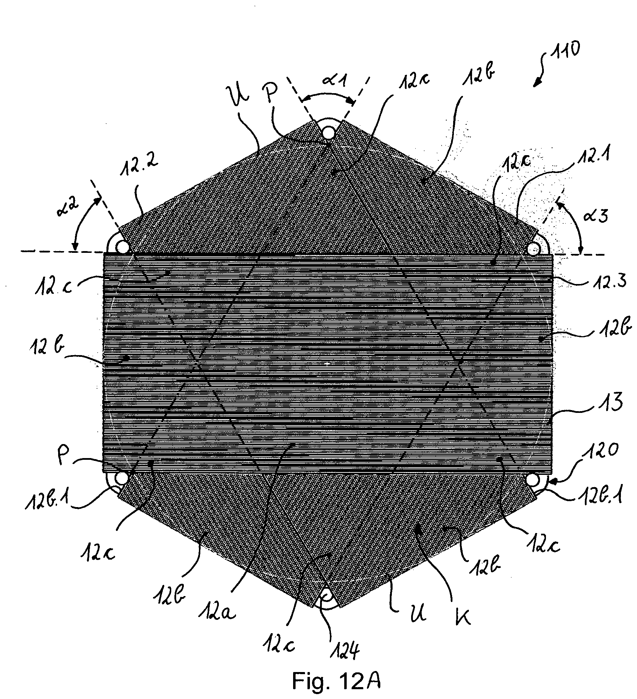

In der Fig. 12A ist ein Oxygenatormodul 110 oder zumindest Komponenten davon gezeigt, welches in Verbindung mit dem in Fig. 11 gezeigten Deckel 120 und Verteiler 140 eingesetzt werden kann. Das Oxygenatormodul 110 weist eine Vielzahl einzelner Hohlfaserlagen auf, von denen hier beispielhaft eine erste Hohlfaserlage 12.1, eine zweite Hohlfaserlage 12.2 und eine dritte Hohlfaserlage 12.3 dargestellt sind. Jede Hohlfaserlage weist eine Vielzahl von linear in einer Richtung ausgerichteten Fasern 13 auf. Die erste Hohlfaserlage 12.1 ist dabei zuunterst angeordnet. Auf der ersten Hohlfaserlage 12.1 ist die zweite Hohlfaserlage 12.2 angeordnet, und auf der zweiten Hohlfaserlage 12.2 ist die dritte Hohlfaserlage 12.3 angeordnet. Die jeweiligen Hohlfaserlagen sind in einem Winkel verdreht zueinander angeordnet. Die erste Hohlfaserlage 12.1 ist in einem Verdrehwinkel α1 zur zweiten Hohlfaserlage 12.2 angeordnet. Die zweite Hohlfaserlage 12.2 ist in einem Verdrehwinkel α2 zur dritten Hohlfaserlage 12.3 angeordnet. Die dritte Hohlfaserlage 12.3 ist in einem Verdrehwinkel α3 zur ersten Hohlfaserlage 12.1 angeordnet. Bevorzugt betragen die Verdrehwinkel jeweils zumindest annähernd 60 Grad. Bevorzugt sind die Verdrehwinkel genau gleich groß. Bei Verdrehwinkeln von exakt 60 Grad kann jeweils nach drei Lagen wieder dieselbe relative Anordnung weiterer Lagen sichergestellt werden, so dass jede der Lagen auf dieselbe Weise umströmbar ist.

Die Hohlfaserlagen sind auf einem hexagonalen Deckel 120 angeordnet, an welchem an einer jeweiligen Ecke ein Ausrichtungselement 124, insbesondere ein Zentrierstift angeordnet ist, mittels welchem die Lagen 12.1, 12.2, 12.3 relativ zum Deckel 120 positionierbar sind. Dabei kann das Ausrichtungselement 124 auch zum relativen Positionieren der gegenüberliegenden Deckel 120 in einem vorbestimmten Abstand zueinander dienen, insbesondere beim Verguss, also beim Ausbilden der Verpottung. Das Ausrichtungselement 124 kann dabei auch die Funktion eines Abstandshalters erfüllen.

In Bezug auf die drei Lagen 12.1, 12.2, 12.3 ergeben sich durch die jeweils um 60 Grad versetzte Anordnung drei unterschiedliche Bereiche oder Abschnitte. In einem Kernbereich 12a überlappen sich alle drei Lagen. Der Kernbereich 12a weist eine hexagonale Grundform auf. In einem jeweiligen hervorstehenden Bereich oder Abschnitt 12b überlappt keine der drei Lagen die anderen beiden Lagen. Insgesamt werden sechs solcher nicht überlappenden, freiliegenden Abschnitte 12b gebildet. Die nicht überlappenden, freiliegenden Abschnitte 12b weisen jeweils eine dreiecksförmige Geometrie mit sich nach radial außen hin daran anschließendem rechteckigem Abschnitt auf. Ferner werden auch teilweise überlappende Abschnitte 12c gebildet, in welchen zwei der drei Lagen einander überlappen. Die teilweise überlappenden Abschnitte 12c weisen eine dreiecksförmige Geometrie auf.

Die nicht überlappenden, freiliegenden Abschnitte 12b weisen jeweils freiliegende Seitenkantenabschnitte 12b.1 auf, mittels welchen eine jeweilige Lage am jeweiligen Ausrichtungselement 124 zur Anlage kommt. Das Oxygenatormodul 110 bzw. die drei Lagen 12.1, 12.2, 12.3 und der Deckel 120 sind zumindest annähernd deckungsgleich ausgebildet. In einer Draufsicht nehmen die drei Lagen 12.1, 12.2, 12.3 und der Deckel 120 zumindest annähernd dieselbe Grundfläche ein. Dabei ist die Länge der Lagen nach einem Bearbeitungsschritt, insbesondere nach einem Schneiden dargestellt. Vor dem Bearbeitungsschritt können die Lagen länger sein.

In der Fig. 12A ist ferner eine Umfangslinie U angedeutet, welche eine Außenmantelfläche einer Verpottung (nicht dargestellt) markiert, insbesondere einen minimalen Durchmesser der Außenmantelfläche. Die Umfangslinie U bzw. die Verpottung umgrenzt eine Kavität K, in welcher die Hohlfaserlagen 12.1, 12.2, 12.3 im Wesentlichen angeordnet sind, und welche von einem Fluid durchströmbar ist. Bei der Herstellung des Oxygenatormoduls 110 kann eine Sperrflüssigkeit verwendet werden, welche aufgrund einer Zentrifugalkraft nach außen getrieben wird. Über die Menge der Sperrflüssigkeit kann die Position der Außenmantelfläche der Verpottung definiert werden. Die Umfangslinie U ist kreisförmig, wobei der Durchmesser der Umfangslinie U zumindest annähernd dem Abstand gegenüberliegender Faserenden entspricht. Bevorzugt ist der Durchmesser maximal so groß wie der Abstand, weiter bevorzugt etwas kleiner als der Abstand, so dass alle Faserenden aus der Verpottung hervorragen und freiliegen. Indem der Durchmesser möglichst (fast) genau so groß ist wie der Abstand, kann das Fasermaterial besonders effektiv ausgenutzt werden. Gemäß einer Variante (wie dargestellt) schneidet die Umfangslinie U eine jeweilige Seitenkante einer jeweiligen Lage in einem Punkt P, in welchem sich auch die Seitenkanten benachbarter Lagen schneiden. Durch diese Anordnung der Verpottung kann ein besonders vorteilhafter Kompromiss beim Ausnutzen des zur Verfügung stehenden Volumens und der nutzbaren Faseroberfläche sichergestellt werden, insbesondere in Verbindung mit der um 60 Grad gedrehten Anordnung der Lagen.

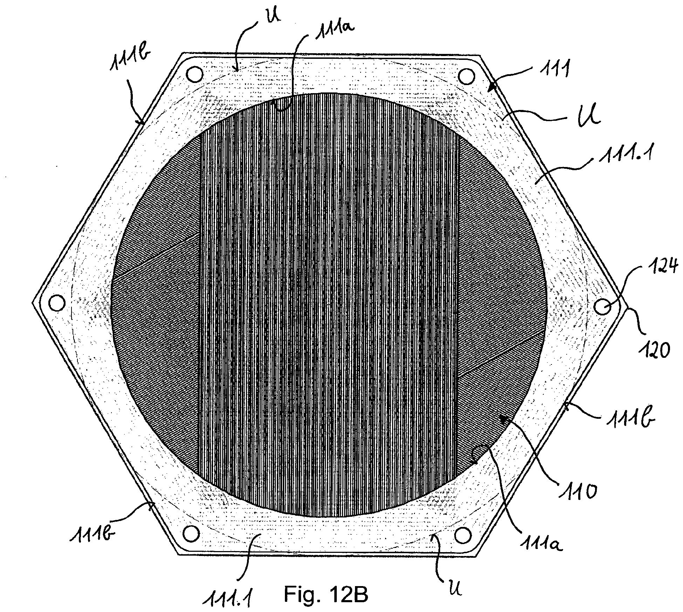

In der Fig. 12B sind im Wesentlichen dieselben Komponenten wie in Fig. 12A gezeigt. Zusätzlich ist die Verpottung 111 gezeigt, aus welcher Ausrichtungselementen 124 hervorstehen. Die Verpottung 111 ist auf einem Deckel 120 angeordnet. Die Verpottung 111 weist einen ringförmigen Abschnitt 111.1 auf, in welchem das Oxygenatormodul 110 eingebettet ist. Der ringförmige Abschnitt 111.1 wird innen durch die Innenmantelfläche 111a der Verpottung und außen durch die Umfangslinie U begrenzt. Die Verpottung 111 weist eine Außenmantelfläche 111b auf, welche im Querschnitt eine hexagonale Geometrie aufweist. Es ist erkennbar, dass die Verpottung 111 nur in einem vergleichsweise kleinen Bereich nicht genutzt wird zum Einbetten der Faserlagen, nämlich in einem Bereich jeweils radial außen von den Ausrichtungselementen 124.

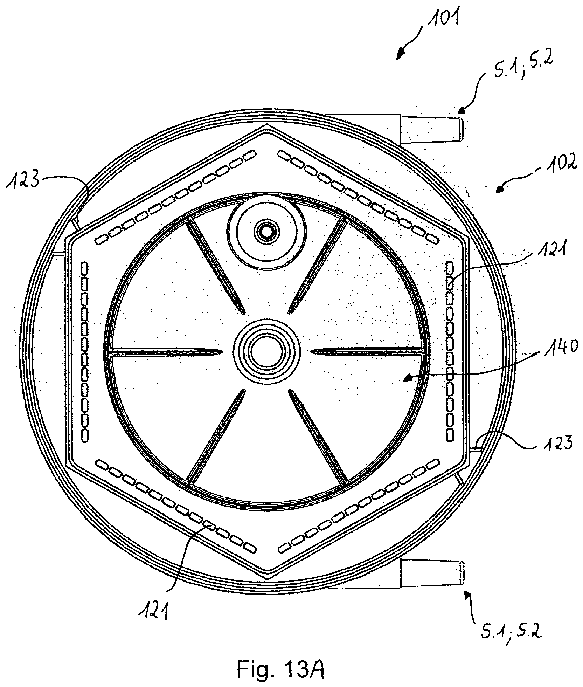

In der Fig. 13A ist ein Oxygenator 101 mit einem (Außen-)Gehäuse 102 gezeigt, welches einen zusätzlichen Fluideinlass 5.1 (insbesondere Gaseinlass) und einen zusätzlichen Fluidauslass 5.2 (insbesondere Gasauslass) aufweist. Ein Deckel 120, z.B. der in der Figur 11 gezeigte Deckel, ist im Gehäuse 102 angeordnet und an einer Innenmantelfläche des Gehäuses mittels Befestigungsmitteln 123 abgestützt. Ein im Oxygenator 101 angeordnetes Oxygenatormodul kann dabei mit zwei Deckeln im Außengehäuse 102 eingesetzt und abgestützt werden. Ferner kann ein zusätzlicher Deckel (nicht dargestellt) vorgesehen sein, welcher das Außengehäuse 102 luftdicht abschließt.

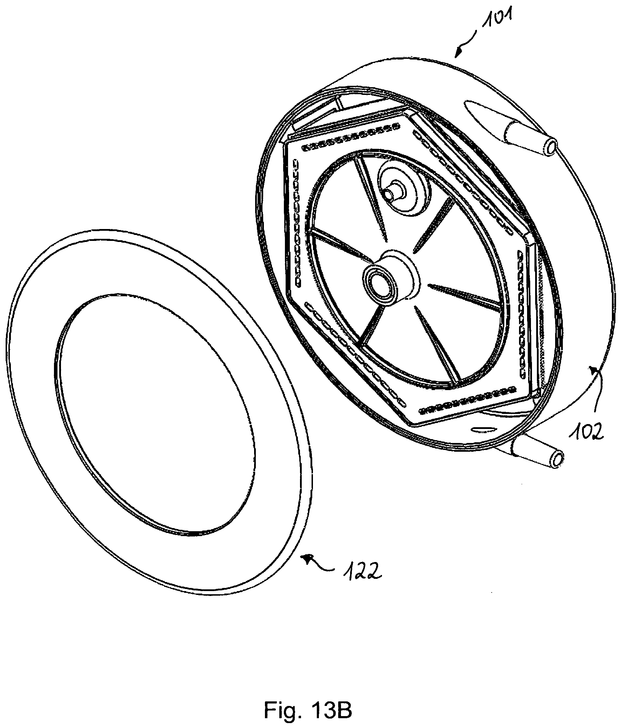

In der Fig. 13B ist ein zusätzlicher Deckel 122 zum Abdecken des (Außen-)Gehäuses 102 gezeigt. Der Deckel 122 weist die Form eines scheibenförmigen Rings auf.

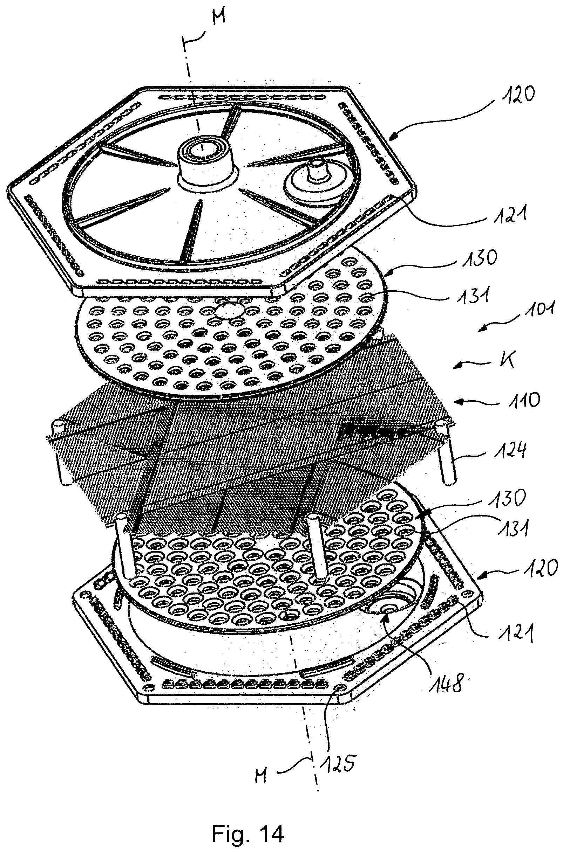

In der Fig. 14 ist ein Oxygenator 101 mit zwei hexagonalen Deckeln 120, Ausrichtungselementen 124, einem hexagonalen Oxygenatormodul 110 sowie zwei kreisrunden Verteilelementen 130 (Blutverteilerplatte, welche geometrisch korrespondierend zur Kavität ausgebildet ist) gezeigt. Die Deckel 120 können identisch aufgebaut sein, was die Symmetrie der Anordnung erhöht und die Anzahl der Komponenten reduzieren kann. Die Deckel 120 umgrenzen im zusammengebauten Zustand eine Kavität K, welche sich entlang der angedeuteten Mittenlängsachse M des Oxygenators 101 bzw. Oxygenatormoduls 110 erstreckt. Der untere Deckel 120 weist einen Auslass 148 bzw. eine rückseitige Entlüftung auf. Ferner ist ein nicht sichtbarer, zentrisch angeordneter Blutauslass vorgesehen. Der Deckel 121 weist Ausnehmungen 125 zur Aufnahme eines jeweiligen Ausrichtungselements 124 auf. Die beiden Verteilelemente 130 sind beidseitig vom Oxygenatormodul 110 angeordnet. Wahlweise kann auch nur ein einzelnes Verteilelement 130 vorgesehen sein, insbesondere auf der Anströmseite. Jedes Verteilelement 130 weist eine Vielzahl von Löchern oder Durchlässen 131 auf, welche zumindest annähernd gleichförmig am Verteilelement 130 verteilt angeordnet sind. Wie dargestellt, können die Durchlässe 131 auf unterschiedlichen Teilkreisen konzentrisch zu einem Mittelpunkt des Verteilelements 130 angeordnet sein. Alle Durchlässe 131 weisen zumindest annähernd denselben Abstand zueinander auf. Die Verteilelemente 130 sind scheibenartig ausgebildet. Mittels des jeweiligen Verteilelements 130 kann ein Fluidstrom flächig auf die gesamte Querschnittsfläche der Kavität K verteilt werden.

### Bezugszeichenliste

- 1; 101: Oxygenator
- 2; 102: Gehäuse
- 4.1: Bluteinlass
- 4.2: Blutauslass
- 5.1: (zusätzlicher) Fluideinlass, insbesondere Gaseinlass
- 5.2: (zusätzlicher) Fluidauslass, insbesondere Gasauslass

- 10; 110: Oxygenatormodul
- 10': Oxygenatormodul gemäß dem Stand der Technik
- 11; 111: Verpottung bzw. Verpottungsmasse
- 111.1: ringförmiger Abschnitt
- 11': Verpottung bei einem Oxygenatormodul gemäß dem Stand der Technik
- 11a; 111a: Innenmantelfläche der Verpottung
- 11b; 111b: Außenmantelfläche der Verpottung
- 12: Hohlfaserlage
- 12.1: erste Hohlfaserlage
- 12.2: zweite Hohlfaserlage
- 12.3: dritte Hohlfaserlage
- 12a: überlappender Kernbereich
- 12b: hervorstehender, nicht überlappender Abschnitt
- 12b.1: freiliegender Seitenkantenabschnitt
- 12c: teilweise überlappender Abschnitt
- 13: Hohlfaser
- 13.1: (erstes) freies Ende einer Hohlfaser
- 13.2: (zweites) freies Ende einer Hohlfaser
- 14: Kettfaden
- 16: Hohlfasermatte
- 16a: Leerstelle
- 16b: Hohlfaserpaket
- 17: Hohlfaserbündel
- 17': Hohlfaserbündel bei einem Oxygenatormodul gemäß dem Stand der Technik

- 20; 120: Deckel
- 21; 121: Befestigungsmittel am Deckel
- 122: zusätzlicher Deckel zum Abdecken des (Außen-)Gehäuses
- 123: Befestigungsmittel zwischen Deckel und Gehäuse
- 124: Abstandshalter oder Ausrichtungselement, insbesondere Zentrierstift
- 125: Ausnehmung zur Aufnahme des Ausrichtungselements
- 30: Drallverteiler30a Innenmantelfläche
- 130: Verteilelement, insbesondere Verteilerplatte
- 131: Loch oder Durchlass

- 30b: Prallfläche
- 31.1: (erstes) Drallelement
- 31.2: (zweites) Drallelement
- 31.3: (drittes) Drallelement
- 31.4: (viertes) Drallelement
- 40: Aspektverteiler
- 140: Verteiler oder Verteilerabschnitt am Deckel
- 40a: Innenmantelfläche
- 40a.1: dritte Innenmantelfläche
- 40a.2: dritte Innenmantelfläche
- 40a.3: dritte Innenmantelfläche
- 41: Blende
- 45: Tangentialverteiler
- 45a: Innenmantelfläche
- 46: Blende
- 47: tangentialer Einlass
- 147: seitlicher Einlass, insbesondere Entlüftung
- 148: seitlicher Auslass, insbesondere Entlüftung

- 50: Form, insbesondere Negativform
- 50': Form für ein Oxygenatormodul gemäß dem Stand der Technik
- 60: Schneideinrichtung

- B: Blut bzw. Blutstrom
- D: Drehachse
- F: Sperrflüssigkeit
- K: Kavität
- M: Mittenlängsachse
- P: Schnittpunkt zwischen Umfangslinie und Seitenkante einer Lage
- U: Umfangslinie

- α1: Verdrehwinkel um Mittenlängsachse zwischen einer ersten und zweiten Lage
- α2: Verdrehwinkel um Mittenlängsachse zwischen einer zweiten und dritten Lage
- α3: Verdrehwinkel um Mittenlängsachse zwischen dritter und erster Lage

## Patentansprüche

1. Oxygenator (1; 101) zum Gasaustausch zwischen Blut und einem Gas in einem extrakorporalen Lungenunterstützungssystem, mit
- mindestens einem Oxygenatormodul (10; 110), mit
• mehreren Lagen (12) von semipermeablen vom Gas durchströmbaren Hohlfasern (13), wobei die Hohlfasern einer der Lagen in einem Verdrehwinkel um eine Mittenlängsachse (M) des Oxygenatormoduls (10; 110) zu den Hohlfasern (13) einer anderen der Lagen ausgerichtet sind; und
• einer Verpottung (11), welche sich entlang der Mittenlängsachse (M) erstreckt und in welcher die Hohlfasern (13) fixiert sind, wobei die Verpottung eine sich entlang der Mittenlängsachse (M) erstreckende Kavität (K) festlegt, in welcher die Hohlfasern (13) angeordnet sind und welche vom Blut in Richtung der Mittenlängsachse (M) durchströmbar ist; wobei die Verpottung (11) eine im Wesentlichen kreisförmige Innenmantelfläche (11a) aufweist, welche die Kavität (K) radial nach außen begrenzt;
- einem Bluteinlass (4.1) und einem Blutauslass (4.2), jeweils gekoppelt mit der blutdurchströmbaren Kavität (K) des Oxygenatormoduls (10; 110); und
- einem Gehäuse (2; 102) zur Aufnahme des Oxygenatormoduls (10; 110), wobei das Gehäuse (2; 102) einen Gaseinlass und einen Gasauslass aufweist, welche jeweils mit Hohlfasern (13) des Oxygenatormoduls gekoppelt sind,
wobei der Oxygenator
- eine Verteilereinrichtung (30; 40; 45; 130) aufweist, welche in Bezug auf die Mittenlängsachse (M) des Oxygenatormoduls (10; 110) stromauf von dem mindestens einen Oxygenatormodul (10; 110) und stromab von dem Bluteinlass (4.1) angeordnet ist, wobei der Oxygenator für eine zentrische Anströmung des Oxygenatormoduls eingerichtet ist,
**dadurch gekennzeichnet, dass** die Verteilereinrichtung einen Drallverteiler (30) aufweist und dazu ausgebildet ist, einen Blutstrom (B) mit einem Drall in einem Strömungswinkel zu der Mittenlängsachse (M) in die Kavität (K) zu lenken.

2. Oxygenator (1) nach Anspruch 1, **dadurch gekennzeichnet, dass** der Bluteinlass und/oder der Blutauslass zentrisch in Bezug auf das Oxygenatormodul angeordnet ist, oder dass die Verteilereinrichtung (30; 40; 45) den Bluteinlass aufweist und derart am Oxygenatormodul angeordnet ist, dass das Oxygenatormodul zentrisch anströmbar ist.

3. Oxygenator (1; 101) nach Anspruch 1 oder 2, **dadurch gekennzeichnet, dass** der Drallverteiler (30) eine im Querschnitt kreisförmige Innenmantelfläche (30a) sowie innenliegende Drallelemente (31.1, 31.2, 31.3, 31.4) aufweist, welche zu einem Mittelpunkt des Drallverteilers (30) hin ineinander zusammenlaufen, und wobei der Drallverteiler (30) bevorzugt Drallelemente (31.1, 31.2, 31.3, 31.4) in Form von Flügeln aufweist, insbesondere vier Flügel.

4. Oxygenator (1; 101) nach einem der Ansprüche 1 bis 3, **dadurch gekennzeichnet, dass** der Oxygenator (1; 101) mindestens einen Deckel (120) aufweist, welcher mittels einer/der Verpottung des Oxygenatormodul fixiert ist, und welcher bevorzugt eine hexagonale Außengeometrie aufweist.

5. Oxygenator (1; 101) nach einem der Ansprüche 1 bis 4, **dadurch gekennzeichnet, dass** die Verteilereinrichtung einen Aspektverteiler (40) oder einen Tangentialverteiler (45) oder einen Verteiler (140) und/oder ein Verteilelement (130) mit einer Vielzahl von Durchlässen (131) jeweils für eine Aufweitung des Blutstroms (B) aufweist.

6. Oxygenator (1; 101) nach Anspruch 1, **dadurch gekennzeichnet, dass** die Verpottung (11) des Oxygenatormoduls (10; 110) eine insbesondere zylindrische Außenmantelfläche (11b) aufweist, aus welcher die Hohlfasern (13) mit mindestens einem freien Ende (13.1, 13.2) radial hervorstehen, wobei die Hohlfasern (13) einer jeweiligen Lage (12) bevorzugt zwei freie Enden (13.1, 13.2) aufweisen und mit beiden freien Enden aus der Verpottung (11) hervorstehen.

7. Oxygenator (1; 101) nach einem der Ansprüche 1 und 6, **dadurch gekennzeichnet, dass** das Oxygenatormodul (110) eine Außengeometrie mit mehr als vier Ecken aufweist, insbesondere eine hexagonale oder eine oktagonale Außengeometrie.

8. Oxygenator (1; 101) nach einem der Ansprüche 1 und 6 bis 7, **dadurch gekennzeichnet, dass** mindestens zwei, bevorzugt mindestens drei, der Lagen (12) des Oxygenatormoduls (10; 110) in einem Winkel ungleich Null und kleiner als 90 Grad verdreht zueinander, insbesondere verdreht um die Mittenlängsachse (M), angeordnet sind, bevorzugt in einem Winkel von zumindest annähernd 45 oder 60 Grad verdreht zueinander.

9. Oxygenator (1; 101) nach einem der Ansprüche 1 und 6 bis 8, **dadurch gekennzeichnet, dass** die Lagen des Oxygenatormoduls (10; 110) eine rechteckige Grundform mit unterschiedlicher Seitenlänge aufweisen.

## Claims

1. Oxygenator (1; 101) for the gas exchange between blood and a gas in an extracorporeal lung support system, with
- at least one oxygenator module (10; 110), with
• several layers (12) of semipermeable, gas-perfusable hollow fibers (13), with the hollow fibers of one of the layers being oriented at an angle of rotation about a central longitudinal axis (M) of the oxygenator module (10; 110) with respect to the hollow fibers (13) of another one of the layers; and
• a potting (11), which extends along the central longitudinal axis (M) and in which hollow fibers (13) are fixed, wherein the potting defines a cavity (K) that extends along the central longitudinal axis (M) and in which hollow fibers (13) are arranged and which is blood-perfusable in the direction of the central longitudinal axis (M), whereby the potting (11) has an essentially circular inner sheath surface (11a) which limits the cavity (K) radially outward;
- a blood inlet (4.1) and a blood outlet (4.2), respectively coupled with a blood-perfusable cavity (K) of the oxygenator module (10; 110); and
- a housing (2; 102) to accommodate the oxygenator module (10; 110), wherein the housing (2; 102) has a gas inlet and a gas outlet, each of which is coupled to the hollow fibers (13) of the oxygenator module,
whereby the oxygenator has
- a distributor device (30; 40; 45; 130) which is arranged, with respect to the central longitudinal axis (M) of the oxygenator module (10; 110), upstream of the at least one oxygenator module (10; 110) and downstream of the blood inlet (4.1), wherein the oxygenator is set up for a central inflow of the oxygenator module,
**characterized in that**
the distributor device has a swirl distributor (30) and is designed to guide a blood stream (B) with a swirl at a flow angle to the central longitudinal axis (M) into the cavity (K).

2. Oxygenator (1) according to claim 1, **characterized in that** the blood inlet and/or the blood outlet are arranged centrally with respect to the oxygenator module, or that the distributor device (30; 40; 45) has a blood inlet and is arranged on the oxygenator module in such a way that the oxygenator module can be fed centrally.

3. Oxygenator (1; 101) according to claim 1 or 2, **characterized in that** the swirl distributor (30) having an inner sheath surface (30a) with a circular cross section as well as internal swirl elements (31.1, 31.2, 31.3, 31.4) that merge into one another toward a central point of the swirl distributor (30) and whereby the swirl distributor (30) preferably having swirl elements (31.1, 31.2, 31.3, 31.4) in the form of wings, particularly four wings.

4. Oxygenator (1; 101) according to one of claims 1 to 3, **characterized in that** the oxygenator (1; 101) has at least one cover (120) which is fixed by means of a/the potting of the oxygenator module and which preferably has a hexagonal outer geometry.

5. Oxygenator (1; 101) according to one of claims 1 to 4, **characterized in that** the distributor device has an aspect distributor (40) or a tangential distributor (45) or a distributor (140) and/or a distribution element (130) with a plurality of passages (131) for an expansion of the blood stream (B) respectively.

6. Oxygenator (1; 101) according to claim 1, **characterized in that** the potting (11) of the oxygenator module (10; 110) has an in particular cylindrical outer sheath surface (11b) from which the hollow fibers (13) radially protrude with at least one free end (13.1, 13.2), wherein the hollow fibers (13) of each respective layer (12) preferably have two free ends (13.1, 13.2) and protrude from the potting (11) with both free ends.

7. Oxygenator (1; 101) according to one of claims 1 to 6, **characterized in that** the oxygenator module (110) has an outer geometry with more than four corners, in particular a hexagonal or a octagonal outer geometry.

8. Oxygenator (1; 101) according to one of claims 1 and 6 to 7, **characterized in that** at least two, preferable at least three, of the layers (12) of the oxygenator module (10; 110) are arranged rotated at an angle unequal to zero and smaller than 90 degrees to one another, particularly rotated about the central longitudinal axis (M), preferably at an angle of at least approximately 45 or 60 degrees.

9. Oxygenator (1; 101) according to one of claims 1 and 6 to 8, **characterized in that** the layers of the oxygenator module (10; 110) have a rectangular basic shape with different lateral lengths.

## Revendications

1. Dispositif d'oxygénation du sang (1; 101) destiné à des échanges gazeux entre du sang et un gaz dans un système d'assistance pulmonaire externe, qui comprend :
- au moins un module (10 ; 110) du dispositif d'oxygénation du sang, comprenant :
• plusieurs couches (12) réalisées à partir de fibres creuses semiperméables (13) qui peuvent être traversées par du gaz ; dans lequel les fibres creuses d'une des couches sont orientées en formant un angle de torsion autour d'un axe longitudinal médian (M) du module (10 ; 110) du dispositif d'oxygénation du sang dans la direction des fibres creuses (13) d'une autre couche parmi lesdites couches ; et
• un remplissage (11) qui s'étend le long de l'axe longitudinal médian (M) et dans lequel sont fixées les fibres creuses (13) ; dans lequel le remplissage établit une cavité (K) qui s'étend le long de l'axe longitudinal médian (M), dans laquelle sont disposées les fibres creuses (13) et qui peut être traversée par le sang dans la direction de l'axe longitudinal médian (M) ; dans lequel le remplissage (11) présente une surface latérale interne (11a) essentiellement de forme circulaire qui délimite la cavité (K) vers l'extérieur en direction radiale ;
- une entrée pour le sang (4.1) et une sortie pour le sang (4.2), respectivement couplées à la cavité (K) du module (10 ; 110) du dispositif d'oxygénation du sang, qui peut être traversée par le sang ; et
- un boîtier (2 ; 102) dans lequel doit venir se loger le module (10 ; 110) du dispositif d'oxygénation du sang ; dans lequel le boîtier (2 ; 102) présente une entrée pour le gaz et une sortie pour le gaz, qui sont respectivement couplées à des fibres creuses (13) du module du dispositif d'oxygénation du sang ;
dans lequel le dispositif d'oxygénation du sang
- présente un mécanisme de répartition (30 ; 40 ; 45 ; 130) qui est disposé, par rapport à l'axe longitudinal médian (M) du module (10 ; 110) du dispositif d'oxygénation du sang, en amont dudit au moins un module (10 ; 110) du dispositif d'oxygénation du sang et en aval de l'entrée pour le sang (4.1) ; dans lequel le dispositif d'oxygénation du sang est conçu pour un afflux dans le module du dispositif d'oxygénation du sang, en position centrale ;
**caractérisé en ce que** le mécanisme de répartition présente un répartiteur de tourbillon (30) et est réalisé dans le but de diriger un flux de sang (B) présentant un tourbillon dans la cavité (K) en formant un angle de flux par rapport à l'axe longitudinal médian (M).

2. Dispositif d'oxygénation du sang (1) selon la revendication 1, **caractérisé en ce que** l'entrée pour le sang et/ou la sortie pour le sang est/sont disposée(s) en position centrale par rapport au module du dispositif d'oxygénation du sang, ou bien **en ce que** le mécanisme de répartition (30 ; 40 ; 45) présente l'entrée pour le sang et est disposé contre le module du dispositif d'oxygénation du sang d'une manière telle que la pénétration du flux dans le module du dispositif d'oxygénation du sang peut se faire en position centrale.

3. Dispositif d'oxygénation du sang (1 ; 101) selon la revendication 1 ou 2, **caractérisé en ce que** le répartiteur de tourbillon (30) présente une surface latérale interne (30a) de forme circulaire en section transversale, ainsi que des éléments giratoires internes (31.1, 31.2, 31.3, 31.4) qui convergent les uns dans les autres à un point central du répartiteur de tourbillon (30) ; et dans lequel le répartiteur de tourbillon (30) présente de préférence des éléments giratoires (31.1, 31.2, 31.3, 31.4) sous la forme d'ailettes, en particulier de quatre ailettes.

4. Dispositif d'oxygénation du sang (1 ; 101) selon l'une quelconque des revendications 1 à 3, **caractérisé en ce que** le dispositif d'oxygénation du sang (1 ; 101) présente au moins un couvercle (120) qui est fixé au moyen d'un/du remplissage du module du dispositif d'oxygénation du sang et qui présente de préférence une géométrie externe de forme hexagonale.

5. Dispositif d'oxygénation du sang (1 ; 101) selon l'une quelconque des revendications 1 à 4, **caractérisé en ce que** le mécanisme de répartition présente un répartiteur d'aspect (40) ou un répartiteur tangentiel (45) ou bien un répartiteur (140) et/ou un élément de répartition (130) comprenant une multitude de passages (131) respectivement destinés à un élargissement du flux sanguin (B).

6. Dispositif d'oxygénation du sang (1 ; 101) selon la revendication 1, **caractérisé en ce que** le remplissage (11) du module (10 ; 110) du dispositif d'oxygénation du sang présente une surface latérale externe (11b) en particulier cylindrique, à partir de laquelle les fibres creuses (13) font saillie en direction radiale avec au moins une extrémité libre (13.1, 13.2) ; dans lequel les fibres creuses (13) d'une couche respective (12) présentent de préférence deux extrémités libres (13.1, 13.2) et font saillie hors du remplissage (11) avec les deux extrémités libres.

7. Dispositif d'oxygénation du sang (1 ; 101) selon l'une quelconque des revendications 1 et 6, **caractérisé en ce que** le module (110) du dispositif d'oxygénation du sang présente une géométrie externe qui comprend plus de quatre coins, en particulier une géométrie externe hexagonale ou une géométrie externe octogonale.

8. Dispositif d'oxygénation du sang (1 ; 101) selon l'une quelconque des revendications 1 et 6 à 7, **caractérisé en ce qu'**au moins deux, de préférence au moins trois couches parmi les couches (12) du module (10 ; 110) du dispositif d'oxygénation du sang sont disposées en torsion en formant un angle qui n'est pas égal à zéro et qui est inférieur à 90 degrés, en particulier en torsion autour de l'axe longitudinal médian (M), de préférence en torsion réciproque en formant un angle d'au moins approximativement 45 ou 60 degrés.

9. Dispositif d'oxygénation du sang (1 ; 101) selon l'une quelconque des revendications 1 et 6 à 8, **caractérisé en ce que** les couches du module (10 ; 110) du dispositif d'oxygénation du sang présentent une forme de base de configuration rectangulaire avec des longueurs de côtés différentes.
